# EUROPÄISCHE PATENTSCHRIFT

(11) **EP 0 972 057 B1**
(45) Veröffentlichungstag und Bekanntmachung des Hinweises auf die Patenterteilung: **26.07.2006**
(21) Anmeldenummer: 98919205.9
(22) Anmeldetag: 02.04.1998
(51) Int. Cl.: C12N 15/81, C12Q 1/68

(54) **VERFAHREN ZUM SCREENING VON ANTIMYKOTISCH WIRKENDEN SUBSTANZEN**
METHOD FOR SCREENING ANTIMYCOTICALLY ACTIVE SUBSTANCES
PROCEDE DE DEPISTAGE DE SUBSTANCES A ACTION ANTIMYCOSIQUE

(30) Priorität: 02.04.1997 DE 19713572
(43) Veröffentlichungstag der Anmeldung: 19.01.2000
(73) Patentinhaber: Sanofi-Aventis Deutschland GmbH, 65929 Frankfurt am Main (DE)
(72) Erfinder: HINNEN, Albert, D-07745 Jena (DE); HEGEMANN, Johannes, D-40589 Düsseldorf (DE); MUNDER, Thomas, D-07743 Jena (DE); SCHUSTER, Tilmer, D-97440 Werneck (DE); FELDMANN, Horst, D-80999 München (DE); KRAMER, Wilfried, D-37077 Göttingen (DE); ZIMMERMANN, Friedrich, Karl, D-64372 Ober-Ramstadt (DE); ENTIAN, Karl-Dieter, D-61440 Oberursel (DE); ROSE, Matthias, D-61267 Neu-Anspach (DE); KÖTTER, Peter, D-61440 Oberursel (DE)
(86) Internationale Anmeldenummer: PCT/EP1998/001904
(87) Internationale Veröffentlichungsnummer: WO 1998/044135

(56) Entgegenhaltungen:
- EP-A- 0 627 491
- EP-A- 0 816 511
- WO-A-95/06132
- WO-A-95/11969
- WO-A-95/34678
- US-A- 5 614 377
- GÜLDENER U. ET AL.: "A new efficient gene disruption cassette for repeated use in budding yeast" NUCL. ACIDS. RES., Bd. 24, Nr. 13, 1996, Seiten 2519-2524, XP002074211 in der Anmeldung erwähnt
- TUITE M.F.: "Antifungal drug development: the identification of new targets" TRENDS IN BIOTECHNOLOGY, Bd. 10, Juli 1992, Seiten 235-239, XP002074212
- VAHLENSIECK H F ET AL: "IDENTIFICATION OF THE YEAST ACC1 GENE PRODUCT (ACETYL-COA CARBOXYLASE) AS THE TARGET OF THE POLYKETIDE FUNGICIDE SORAPHEN A" CURRENT GENETICS, Bd. 25, Nr. 2, 1.Februar 1994, Seiten 95-100, XP000196332
- DATABASE SWISS PROT Accession No. P53230, 1.Oktober 1996 ENTIAN K.D. ET AL.: "YGR046W, hypothetical 44.2 kDa protein; from S. cerevisiae Chromosome VII" XP002074214
- MUMBERG D. ET AL.: "Regulatable promoters of S. cerevisiae: comparison of transcriptional activity and their use for heterologous expression" NUCL. ACIDS RES., Bd. 22, Nr. 25, 1994, Seiten 5767-5768, XP002074213 in der Anmeldung erwähnt

### SEQUENZPROTOKOLL

(1) ALLGEMEINE INFORMATION:
   (i) ANMELDER:
      (A) NAME: Hoechst Aktiengesellschaft
      (B) STRASSE: -
      (C) ORT: Frankfurt
      (D) BUNDESLAND: -
      (E) LAND: Deutschland
      (F) POSTLEITZAHL: 65926
      (G) TELEPHON: 069-305-7072
      (H) TELEFAX: 069-35-7175
      (I) TELEX: -
   (ii) ANMELDETITEL: Verfahren zum Screening von antimykotisch wirkenden Substanzen
   (iii) ANZAHL DER SEQUENZEN: 37
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 50 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..50
   (xi) SEQUENZBESCHREIBUNG: SEQ ID NO: 1:
      GAGAGAGAGA GAGAGAGAGA ACTAGXXXXX XTTTTTTTTT TTTTTTTTTT 50
(2) INFORMATION ZU SEQ ID NO: 2:
   (i) SEQUENZ CHARAKTERISTIKA:
      (A) LÄNGE: 13 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..13
   (xi) SEQUENZBESCHREIBUNG: SEQ ID NO: 2:
      XXXXXGGCAC GAG 13
(2) INFORMATION ZU SEQ ID NO: 3:
   (i) SEQUENZ CHARAKTERISTIKA:
      (A) LÄNGE: 24 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..24
   (xi) SEQUENZBESCHREIBUNG: SEQ ID NO: 3:
      GCGGCCGCAA GGCCTCCATG GCCG 24
(2) INFORMATION ZU SEQ ID NO: 4:
   (i) SEQUENZ CHARAKTERISTIKA:
      (A) LÄNGE: 32 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..32
   (xi) SEQUENZBESCHREIBUNG: SEQ ID NO: 4:
      GATCCGGCCA TGGAGGCCTT GCGGCCGCTG CA 32
(2) INFORMATION ZU SEQ ID NO: 5:
   (i) SEQUENZ CHARAKTERISTIKA:
      (A) LÄNGE: 37 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..37
   (xi) SEQUENZBESCHREIBUNG: SEQ ID NO: 5:
      ATCTGCAGCG GCCGCGTTTT AAGAGCTTGG TGAGCGC 37
(2) INFORMATION ZU SEQ ID NO: 6:
   (i) SEQUENZ CHARAKTERISTIKA:
      (A) LÄNGE: 41 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..41
   (xi) SEQUENZBESCHREIBUNG: SEQ ID NO: 6:
      ATGGATCCGG CCATGGAGGC CTCGTTCAGA ATGACACGTA T 41
(2) INFORMATION ZU SEQ ID NO: 7:
   (i) SEQUENZ CHARAKTERISTIKA:
      (A) LÄNGE: 42 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..42
   (xi) SEQUENZBESCHREIBUNG: SEQ ID NO: 7:
      ATGGATCCGG CCATGGAGGC CTGTGGGAAT ACTCAGGTAT CG 42
(2) INFORMATION ZU SEQ ID NO: 8:
   (i) SEQUENZ CHARAKTERISTIKA:
      (A) LÄNGE: 40 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..40
   (xi) SEQUENZBESCHREIBUNG: SEQ ID NO: 8:
      ATCTGCAGCG GCCGCGTCTA CCCTATGAAC ATATTCCATT 40
(2) INFORMATION ZU SEQ ID NO: 9:
   (i) SEQUENZ CHARAKTERISTIKA:
      (A) LÄNGE: 40 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..40
   (xi) SEQUENZBESCHREIBUNG: SEQ ID NO: 9:
      ATCTGCAGCG GCCGCAAACA TGAGAATTGG GTAATAACTG 40
(2) INFORMATION ZU SEQ ID NO: 10:
   (i) SEQUENZ CHARAKTERISTIKA:
      (A) LÄNGE: 47 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..47
   (xi) SEQUENZBESCHREIBUNG: SEQ ID NO: 10:
      ATGGATCCGG CCATGGAGGC CTTCAAGAAT TAGCTTTTCA ATTCATC 47
(2) INFORMATION ZU SEQ ID NO: 11:
   (i) SEQUENZ CHARAKTERISTIKA:
      (A) LÄNGE: 34 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..34
   (xi) SEQUENZBESCHREIBUNG: SEQ ID NO: 11:
      ATCTGCAGCG GCCGCATTTA ATAGAACAGC ATCG 34
(2) INFORMATION ZU SEQ ID NO: 12:
   (i) SEQUENZ CHARAKTERISTIKA:
      (A) LÄNGE: 38 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..38
   (xi) SEQUENZBESCHREIBUNG: SEQ ID NO: 12:
      ATGGATCCGG CCATGGAGGC CACACCGCAT AGATCGGC 38
(2) INFORMATION ZU SEQ ID NO: 13:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..19
   (xi) SEQUENZBESCHREIBUNG: SEQ ID NO: 13:
      AGGCAGACTA CAACTTAGG 19
(2) INFORMATION ZU SEQ ID NO: 14:
   (i) SEQUENZ CHARAKTERISTIKA:
      (A) LÄNGE: 25 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..25
   (xi) SEQUENZBESCHREIBUNG: SEQ ID NO: 14:
      CTGAATTCGA TGAGGAGAAG CTAGT 25
(2) INFORMATION ZU SEQ ID NO: 15:
   (i) SEQUENZ CHARAKTERISTIKA:
      (A) LÄNGE: 23 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..23
   (xi) SEQUENZBESCHREIBUNG: SEQ ID NO: 15:
      CTTCAAACGC TTGTTAAATC TTG 23
(2) INFORMATION ZU SEQ ID NO: 16:
   (i) SEQUENZ CHARAKTERISTIKA:
      (A) LÄNGE: 25 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..25
   (xi) SEQUENZBESCHREIBUNG: SEQ ID NO: 16:
      CAGGATCCGT AGACCATTTT CAGAA 25
(2) INFORMATION ZU SEQ ID NO: 17:
   (i) SEQUENZ CHARAKTERISTIKA:
      (A) LÄNGE: 2973 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..2973
   (xi) SEQUENZBESCHREIBUNG: SEQ ID NO: 17:
   ATTGTAAGCG TTAATATTTT GTTAAAATTC GCGTTAAATT TTTGTTAAAT CAGCTCATTT    60
   TTTAACCAAT AGGCCGAAAT CGGCAAAATC CCTTATAAAT CAAAAGAATA GACCGAGATA   120
   GGGTTGAGTG TTGTTCCAGT TTGGAACAAG AGTCCACTAT TAAAGAACGT GGACTCCAAC   180
   GTCAAAGGGC GAAAAACCGT CTATCAGGGC GATGGCCCAC TACGTGAACC ATCACCCTAA   240
   TCAAGTTTTT TGGGGTCGAG GTGCCGTAAA GCACTAAATC GGAACCCTAA AGGGAGCCCC   300
   CGATTTAGAG CTTGACGGGG AAAGCCGGCG AACGTGGCGA GAAAGGAAGG GAAGAAAGCG   360
   AAAGGAGCGG GCGCTAGGGC GCTGGCAAGT GTAGCGGTCA CGCTGCGCGT AACCACCACA   420
   CCCGCCGCGC TTAATGCGCC GCTACAGGGC GCGTCCCATT CGCCATTCAG GCTGCGCAAC   480
   TGTTGGGAAG GGCGATCGGT GCGGGCCTCT TCGCTATTAC GCCAGCTGGC GAAAGGGGGA   540
   TGTGCTGCAA GGCGATTAAG TTGGGTAACG CCAGGGTTTT CCCAGTCACG ACGTTGTAAA   600
   ACGACGGCCA GTGAGCGCGC GTAATACGAC TCACTATAGG GCGAATTGGA GCTCCACCGC   660
   GGTGGCGCTC TAGAACTAGT GGATCCGGCC ATGGAGGCCT TGCGGCCGCT GCAGGAATTC   720
   GATATCAAGC TTATCGATAC CGTCGACCTC GAGGGGGGGC CCGGTACCCA GCTTTTGTTC   780
   CCTTTAGTGA GGGTTAATTG CGCGCTTGGC GTAATCATGG TCATAGCTGT TTCCTGTGTG   840
   AAATTGTTAT CCGCTCACAA TTCCACACAA CATACGAGCC GGAAGCATAA AGTGTAAAGC   900
   CTGGGGTGCC TAATGAGTGA GCTAACTCAC ATTAATTGCG TTGCGCTCAC TGCCCGCTTT     960
   CCAGTCGGGA AACCTGTCGT GCCAGCTGCA TTAATGAATC GGCCAACGCG CGGGGAGAGG    1020
   CGGTTTGCGT ATTGGGCGCT CTTCCGCTTC CTCGCTCACT GACTCGCTGC GCTCGGTCGT    1080
   TCGGCTGCGG CGAGCGGTAT CAGCTCACTC AAAGGCGGTA ATACGGTTAT CCACAGAATC    1140
   AGGGGATAAC GCAGGAAAGA ACATGTGAGC AAAAGGCCAG CAAAAGGCCA GGAACCGTAA    1200
   AAAGGCCGCG TTGCTGGCGT TTTTCCATAG GCTCCGCCCC CCTGACGAGC ATCACAAAAA    1260
   TCGACGCTCA AGTCAGAGGT GGCGAAACCC GACAGGACTA TAAAGATACC AGGCGTTTCC    1320
   CCCTGGAAGC TCCCTCGTGC GCTCTCCTGT TCCGACCCTG CCGCTTACCG GATACCTGTC    1380
   CGCCTTTCTC CCTTCGGGAA GCGTGGCGCT TTCTCATAGC TCACGCTGTA GGTATCTCAG    1440
   TTCGGTGTAG GTCGTTCGCT CCAAGCTGGG CTGTGTGCAC GAACCCCCCG TTCAGCCCGA    1500
   CCGCTGCGCC TTATCCGGTA ACTATCGTCT TGAGTCCAAC CCGGTAAGAC ACGACTTATC    1560
   GCCACTGGCA GCAGCCACTG GTAACAGGAT TAGCAGAGCG AGGTATGTAG GCGGTGCTAC    1620
   AGAGTTCTTG AAGTGGTGGC CTAACTACGG CTACACTAGA AGGACAGTAT TTGGTATCTG    1680
   CGCTCTGCTG AAGCCAGTTA CCTTCGGAAA AAGAGTTGGT AGCTCTTGAT CCGGCAAACA    1740
   AACCACCGCT GGTAGCGGTG GTTTTTTTGT TTGCAAGCAG CAGATTACGC GCAGAAAAAA    1800
   AGGATCTCAA GAAGATCCTT TGATCTTTTC TACGGGGTCT GACGCTCAGT GGAACGAAAA    1860
   CTCACGTTAA GGGATTTTGG TCATGAGATT ATCAAAAAGG ATCTTCACCT AGATCCTTTT    1920
   AAATTAAAAA TGAAGTTTTA AATCAATCTA AAGTATATAT GAGTAAACTT GGTCTGACAG    1980
   TTACCAATGC TTAATCAGTG AGGCACCTAT CTCAGCGATC TGTCTATTTC GTTCATCCAT    2040
   AGTTGCCTGA CTCCCCGTCG TGTAGATAAC TACGATACGG GAGGGCTTAC CATCTGGCCC    2100
   CAGTGCTGCA ATGATACCGC GAGACCCACG CTCACCGGCT CCAGATTTAT CAGCAATAAA    2160
   CCAGCCAGCC GGAAGGGCCG AGCGCAGAAG TGGTCCTGCA ACTTTATCCG CCTCCATCCA    2220
   GTCTATTAAT TGTTGCCGGG AAGCTAGAGT AAGTAGTTCG CCAGTTAATA GTTTGCGCAA    2280
   CGTTGTTGCC ATTGCTACAG GCATCGTGGT GTCACGCTCG TCGTTTGGTA TGGCTTCATT    2340
   CAGCTCCGGT TCCCAACGAT CAAGGCGAGT TACATGATCC CCCATGTTGT GCAAAAAAGC    2400
   GGTTAGCTCC TTCGGTCCTC CGATCGTTGT CAGAAGTAAG TTGGCCGCAG TGTTATCACT    2460
   CATGGTTATG GCAGCACTGC ATAATTCTCT TACTGTCATG CCATCCGTAA GATGCTTTTC 2520
   TGTGACTGGT GAGTACTCAA CCAAGTCATT CTGAGAATAG TGTATGCGGC GACCGAGTTG 2580
   CTCTTGCCCG GCGTCAATAC GGGATAATAC CGCGCCACAT AGCAGAACTT TAAAAGTGCT 2640
   CATCATTGGA AAACGTTCTT CGGGGCGAAA ACTCTCAAGG ATCTTACCGC TGTTGAGATC 2700
   CAGTTCGATG TAACCCACTC GTGCACCCAA CTGATCTTCA GCATCTTTTA CTTTCACCAG 2760
   CGTTTCTGGG TGAGCAAAAA CAGGAAGGCA AAATGCCGCA AAAAAGGGAA TAAGGGCGAC 2820
   ACGGAAATGT TGAATACTCA TACTCTTCCT TTTTCAATAT TATTGAAGCA TTTATCAGGG 2880
   TTATTGTCTC ATGAGCGGAT ACATATTTGA ATGTATTTAG AAAAATAAAC AAATAGGGGT 2940
   TCCGCGCACA TTTCCCCGAA AAGTGCCACC TAA 2973
(2) INFORMATION ZU SEQ ID NO: 18:
   (i) SEQUENZ CHARAKTERISTIKA:
      (A) LÄNGE: 4088 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..4088
   (xi) SEQUENZBESCHREIBUNG: SEQ ID NO: 18:
   ATTGTAAGCG TTAATATTTT GTTAAAATTC GCGTTAAATT TTTGTTAAAT CAGCTCATTT 60
   TTTAACCAAT AGGCCGAAAT CGGCAAAATC CCTTATAAAT CAAAAGAATA GACCGAGATA 120
   GGGTTGAGTG TTGTTCCAGT TTGGAACAAG AGTCCACTAT TAAAGAACGT GGACTCCAAC 180
   GTCAAAGGGC GAAAAACCGT CTATCAGGGC GATGGCCCAC TACGTGAACC ATCACCCTAA 240
   TCAAGTTTTT TGGGGTCGAG GTGCCGTAAA GCACTAAATC GGAACCCTAA AGGGAGCCCC 300
   CGATTTAGAG CTTGACGGGG AAAGCCGGCG AACGTGGCGA GAAAGGAAGG GAAGAAAGCG 360
   AAAGGAGCGG GCGCTAGGGC GCTGGCAAGT GTAGCGGTCA CGCTGCGCGT AACCACCACA 420
   CCCGCCGCGC TTAATGCGCC GCTACAGGGC GCGTCCCATT CGCCATTCAG GCTGCGCAAC   480
   TGTTGGGAAG GGCGATCGGT GCGGGCCTCT TCGCTATTAC GCCAGCTGGC GAAAGGGGGA   540
   TGTGCTGCAA GGCGATTAAG TTGGGTAACG CCAGGGTTTT CCCAGTCACG ACGTTGTAAA   600
   ACGACGGCCA GTGAGCGCGC GTAATACGAC TCACTATAGG GCGAATTGGA GCTCCACCGC   660
   GGTGGCGCTC TAGAACTAGT GGATCCGGCC ATGGAGGCCT CGTTCAGAAT GACACGTATA   720
   GAATGATGCA TTACCTTGTC ATCTTCAGTA TCATACTGTT CGTATACATA CTTACTGACA   780
   TTCATAGGTA TACATATATA CACATGTATA TATATCGTAT GCTGCAGCTT TAAATAATCG   840
   GTGTCACTAC ATAAGAACAC CTTTGGTGGA GGGAACATCG TTGGTACCAT TGGGCGAGGT   900
   GGCTTCTCTT ATGGCAACCG CAAGAGCCTT GAACGCACTC TCACTACGGT GATGATCATT   960
   CTTGCCTCGC AGACAATCAA CGTGGAGGGT AATTCTGCTA GCCTCTGCAA AGCTTTCAAG  1020
   AAAATGCGGG ATCATCTCGC AAGAGAGATC TCCTACTTTC TCCCTTTGCA AACCAAGTTC  1080
   GACAACTGCG TACGGCCTGT TCGAAAGATC TACCACCGCT CTGGAAAGTG CCTCATCCAA  1140
   AGGCGCAAAT CCTGATCCAA ACCTTTTTAC TCCACGCGCC AGTAGGGCCT CTTTAAAAGC  1200
   TTGACCGAGA GCAATCCCGC AGTCTTCAGT GGTGTGATGG TCGTCTATGT GTAAGTCACC  1260
   AATGCACTCA ACGATTAGCG ACCAGCCGGA ATGCTTGGCC AGAGCATGTA TCATATGGTC  1320
   CAGAAACCCT ATACCTGTGT GGACGTTAAT CACTTGCGAT TGTGTGGCCT GTTCTGCTAC  1380
   TGCTTCTGCC TCTTTTTCTG GGAAGATCGA GTGCTCTATC GCTAGGGGAC CACCCTTTAA  1440
   AGAGATCGCA ATCTGAATCT TGGTTTCATT TGTAATACGC TTTACTAGGG CTTTCTGCTC  1500
   TGTCATCTTT GCCTTCGTTT ATCTTGCCTG CTCATTTTTT AGTATATTCT TCGAAGAAAT  1560
   CACATTACTT TATATAATGT ATAATTCATT ATGTGATAAT GCCAATCGCT AAGAAAAAAA  1620
   AAGAGTCATC CGCTAGGGGA AAAAAAAAAA TGAAAATCAT TACCGAGGCA TAAAAAAATA  1680
   TAGAGTGTAC TAGAGGAGGC CAAGAGTAAT AGAAAAAGAA AATTGCGGGA AAGGACTGTG  1740
   TTATGACTTC CCTGACTAAT GCCGTGTTCA AACGATACCT GGCAGTGACT CCTAGCGCTC  1800
   ACCAAGCTCT TAAAACGCGG CCGCTGCAGG AATTCGATAT CAAGCTTATC GATACCGTCG  1860
   ACCTCGAGGG GGGGCCCGGT ACCCAGCTTT TGTTCCCTTT AGTGAGGGTT AATTGCGCGC  1920
   TTGGCGTAAT CATGGTCATA GCTGTTTCCT GTGTGAAATT GTTATCCGCT CACAATTCCA  1980
   CACAACATAC GAGCCGGAAG CATAAAGTGT AAAGCCTGGG GTGCCTAATG AGTGAGCTAA    2040
   CTCACATTAA TTGCGTTGCG CTCACTGCCC GCTTTCCAGT CGGGAAACCT GTCGTGCCAG    2100
   CTGCATTAAT GAATCGGCCA ACGCGCGGGG AGAGGCGGTT TGCGTATTGG GCGCTCTTCC    2160
   GCTTCCTCGC TCACTGACTC GCTGCGCTCG GTCGTTCGGC TGCGGCGAGC GGTATCAGCT    2220
   CACTCAAAGG CGGTAATACG GTTATCCACA GAATCAGGGG ATAACGCAGG AAAGAACATG    2280
   TGAGCAAAAG GCCAGCAAAA GGCCAGGAAC CGTAAAAAGG CCGCGTTGCT GGCGTTTTTC    2340
   CATAGGCTCC GCCCCCCTGA CGAGCATCAC AAAAATCGAC GCTCAAGTCA GAGGTGGCGA    2400
   AACCCGACAG GACTATAAAG ATACCAGGCG TTTCCCCCTG GAAGCTCCCT CGTGCGCTCT    2460
   CCTGTTCCGA CCCTGCCGCT TACCGGATAC CTGTCCGCCT TTCTCCCTTC GGGAAGCGTG    2520
   GCGCTTTCTC ATAGCTCACG CTGTAGGTAT CTCAGTTCGG TGTAGGTCGT TCGCTCCAAG    2580
   CTGGGCTGTG TGCACGAACC CCCCGTTCAG CCCGACCGCT GCGCCTTATC CGGTAACTAT    2640
   CGTCTTGAGT CCAACCCGGT AAGACACGAC TTATCGCCAC TGGCAGCAGC CACTGGTAAC    2700
   AGGATTAGCA GAGCGAGGTA TGTAGGCGGT GCTACAGAGT TCTTGAAGTG GTGGCCTAAC    2760
   TACGGCTACA CTAGAAGGAC AGTATTTGGT ATCTGCGCTC TGCTGAAGCC AGTTACCTTC    2820
   GGAAAAAGAG TTGGTAGCTC TTGATCCGGC AAACAAACCA CCGCTGGTAG CGGTGGTTTT    2880
   TTTGTTTGCA AGCAGCAGAT TACGCGCAGA AAAAAAGGAT CTCAAGAAGA TCCTTTGATC    2940
   TTTTCTACGG GGTCTGACGC TCAGTGGAAC GAAAACTCAC GTTAAGGGAT TTTGGTCATG    3000
   AGATTATCAA AAAGGATCTT CACCTAGATC CTTTTAAATT AAAAATGAAG TTTTAAATCA    3060
   ATCTAAAGTA TATATGAGTA AACTTGGTCT GACAGTTACC AATGCTTAAT CAGTGAGGCA    3120
   CCTATCTCAG CGATCTGTCT ATTTCGTTCA TCCATAGTTG CCTGACTCCC CGTCGTGTAG    3180
   ATAACTACGA TACGGGAGGG CTTACCATCT GGCCCCAGTG CTGCAATGAT ACCGCGAGAC    3240
   CCACGCTCAC CGGCTCCAGA TTTATCAGCA ATAAACCAGC CAGCCGGAAG GGCCGAGCGC    3300
   AGAAGTGGTC CTGCAACTTT ATCCGCCTCC ATCCAGTCTA TTAATTGTTG CCGGGAAGCT    3360
   AGAGTAAGTA GTTCGCCAGT TAATAGTTTG CGCAACGTTG TTGCCATTGC TACAGGCATC    3420
   GTGGTGTCAC GCTCGTCGTT TGGTATGGCT TCATTCAGCT CCGGTTCCCA ACGATCAAGG    3480
   CGAGTTACAT GATCCCCCAT GTTGTGCAAA AAAGCGGTTA GCTCCTTCGG TCCTCCGATC    3540
   GTTGTCAGAA GTAAGTTGGC CGCAGTGTTA TCACTCATGG TTATGGCAGC ACTGCATAAT 3600
   TCTCTTACTG TCATGCCATC CGTAAGATGC TTTTCTGTGA CTGGTGAGTA CTCAACCAAG 3660
   TCATTCTGAG AATAGTGTAT GCGGCGACCG AGTTGCTCTT GCCCGGCGTC AATACGGGAT 3720
   AATACCGCGC CACATAGCAG AACTTTAAAA GTGCTCATCA TTGGAAAACG TTCTTCGGGG 3780
   CGAAAACTCT CAAGGATCTT ACCGCTGTTG AGATCCAGTT CGATGTAACC CACTCGTGCA 3840
   CCCAACTGAT CTTCAGCATC TTTTACTTTC ACCAGCGTTT CTGGGTGAGC AAAAACAGGA 3900
   AGGCAAAATG CCGCAAAAAA GGGAATAAGG GCGACACGGA AATGTTGAAT ACTCATACTC 3960
   TTCCTTTTTC AATATTATTG AAGCATTTAT CAGGGTTATT GTCTCATGAG CGGATACATA 4020
   TTTGAATGTA TTTAGAAAAA TAAACAAATA GGGGTTCCGC GCACATTTCC CCGAAAAGTG 4080
   CCACCTAA 4088
(2) INFORMATION ZU SEQ ID NO: 19:
   (i) SEQUENZ CHARAKTERISTIKA:
      (A) LÄNGE: 4583 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..4583
   (xi) SEQUENZBESCHREIBUNG: SEQ ID NO: 19:
   ATTGTAAGCG TTAATATTTT GTTAAAATTC GCGTTAAATT TTTGTTAAAT CAGCTCATTT 60
   TTTAACCAAT AGGCCGAAAT CGGCAAAATC CCTTATAAAT CAAAAGAATA GACCGAGATA 120
   GGGTTGAGTG TTGTTCCAGT TTGGAACAAG AGTCCACTAT TAAAGAACGT GGACTCCAAC 180
   GTCAAAGGGC GAAAAACCGT CTATCAGGGC GATGGCCCAC TACGTGAACC ATCACCCTAA 240
   TCAAGTTTTT TGGGGTCGAG GTGCCGTAAA GCACTAAATC GGAACCCTAA AGGGAGCCCC 300
   CGATTTAGAG CTTGACGGGG AAAGCCGGCG AACGTGGCGA GAAAGGAAGG GAAGAAAGCG 360
   AAAGGAGCGG GCGCTAGGGC GCTGGCAAGT GTAGCGGTCA CGCTGCGCGT AACCACCACA 420
   CCCGCCGCGC TTAATGCGCC GCTACAGGGC GCGTCCCATT CGCCATTCAG GCTGCGCAAC 480
   TGTTGGGAAG GGCGATCGGT GCGGGCCTCT TCGCTATTAC GCCAGCTGGC GAAAGGGGGA 540
   TGTGCTGCAA GGCGATTAAG TTGGGTAACG CCAGGGTTTT CCCAGTCACG ACGTTGTAAA 600
   ACGACGGCCA GTGAGCGCGC GTAATACGAC TCACTATAGG GCGAATTGGA GCTCCACCGC 660
   GGTGGCGCTC TAGAACTAGT GGATCCGGCC ATGGAGGCCT GTGGGAATAC TCAGGTATCG 720
   TAAGATGCAA GAGTTCGAAT CTCTTAGCAA CCATTATTTT TTTCCTCAAC ATAACGAGAA 780
   CACACAGGGG CGCTATCGCA CAGAATCAAA TTCGATGACT GGAAATTTTT TGTTAATTTC 840
   AGAGGTCGCC TGACGCATAT ACCTTTTTCA ACTGAAAAAT TGGGAGAAAA AGGAAAGGTG 900
   AGAGGCCGGA ACCGGCTTTT CATATAGAAT AGAGAAGCGT TCATGACTAA ATGCTTGCAT 960
   CACAATACTT GAAGTTGACA ATATTATTTA AGGACCTATT GTTTTTTCCA ATAGGTGGTT 1020
   AGCAATCGTC TTACTTTCTA ACTTTTCTTA CCTTTTACAT TTCAGCAATA TATATATATA 1080
   TTTCAAGGAT ATACCATTCT AATGTCTGCC CCTATGTCTG CCCCTAAGAA GATCGTCGTT 1140
   TTGCCAGGTG ACCACGTTGG TCAAGAAATC ACAGCCGAAG CCATTAAGGT TCTTAAAGCT 1200
   ATTTCTGATG TTCGTTCCAA TGTCAAGTTC GATTTCGAAA ATCATTTAAT TGGTGGTGCT 1260
   GCTATCGATG CTACAGGTGT CCCACTTCCA GATGAGGCGC TGGAAGCCTC CAAGAAGGTT 1320
   GATGCCGTTT TGTTAGGTGC TGTGGGTGGT CCTAAATGGG GTACAGGTAG TGTTAGACCT 1380
   GAACAAGGTT TACTAAAAAT CCGTAAAGAA CTTCAATTGT ACGCCAACTT AAGACCATGT 1440
   AACTTTGCAT CCGACTCTCT TTTAGACTTA TCTCCAATCA AGCCACAATT TGCTAAAGGT 1500
   ACTGACTTCG TTGTTGTCAG AGAATTAGTG GGAGGTATTT ACTTTGGTAA GAGAAAGGAA 1560
   GACGATGGTG ATGGTGTCGC TTGGGATAGT GAACAATACA CCGTTCCAGA AGTGCAAAGA 1620
   ATCACAAGAA TGGCCGCTTT CATGGCCCTA CAACATGAGC CACCATTGCC TATTTGGTCC 1680
   TTGGATAAAG CTAATGTTTT GGCCTCTTCA AGATTATGGA GAAAAACTGT GGAGGAAACC 1740
   ATCAAGAACG AATTTCCTAC ATTGAAGGTT CAACATCAAT TGATTGATTC TGCCGCCATG 1800
   ATCCTAGTTA AGAACCCAAC CCACCTAAAT GGTATTATAA TCACCAGCAA CATGTTTGGT 1860
   GATATCATCT CCGATGAAGC CTCCGTTATC CCAGGTTCCT TGGGTTTGTT GCCATCTGCG 1920
   TCCTTGGCCT CTTTGCCAGA CAAGAACACC GCATTTGGTT TGTACGAACC ATGCCACGGT 1980
   TCTGCTCCAG ATTTGCCAAA GAATAAGGTT GACCCTATCG CCACTATCTT GTCTGCTGCA 2040
   ATGATGTTGA AATTGTCATT GAACTTGCCT GAAGAAGGTA AGGCCATTGA AGATGCAGTT 2100
   AAAAAGGTTT TGGATGCAGG TATCAGAACT GGTGATTTAG GTGGTTCCAA CAGTACCACC 2160
   GAAGTCGGTG ATGCTGTCGC CGAAGAAGTT AAGAAAATCC TTGCTTAAAA AGATTCTCTT 2220
   TTTTTATGAT ATTTGTACAT AAACTTTATA AATGAAATTC ATAATAGAAA CGACACGAAA 2280
   TTACAAAATG GAATATGTTC ATAGGGTAGA CGCGGCCGCT GCAGGAATTC GATATCAAGC 2340
   TTATCGATAC CGTCGACCTC GAGGGGGGGC CCGGTACCCA GCTTTTGTTC CCTTTAGTGA 2400
   GGGTTAATTG CGCGCTTGGC GTAATCATGG TCATAGCTGT TTCCTGTGTG AAATTGTTAT 2460
   CCGCTCACAA TTCCACACAA CATACGAGCC GGAAGCATAA AGTGTAAAGC CTGGGGTGCC 2520
   TAATGAGTGA GCTAACTCAC ATTAATTGCG TTGCGCTCAC TGCCCGCTTT CCAGTCGGGA 2580
   AACCTGTCGT GCCAGCTGCA TTAATGAATC GGCCAACGCG CGGGGAGAGG CGGTTTGCGT 2640
   ATTGGGCGCT CTTCCGCTTC CTCGCTCACT GACTCGCTGC GCTCGGTCGT TCGGCTGCGG 2700
   CGAGCGGTAT CAGCTCACTC AAAGGCGGTA ATACGGTTAT CCACAGAATC AGGGGATAAC 2760
   GCAGGAAAGA ACATGTGAGC AAAAGGCCAG CAAAAGGCCA GGAACCGTAA AAAGGCCGCG 2820
   TTGCTGGCGT TTTTCCATAG GCTCCGCCCC CCTGACGAGC ATCACAAAAA TCGACGCTCA 2880
   AGTCAGAGGT GGCGAAACCC GACAGGACTA TAAAGATACC AGGCGTTTCC CCCTGGAAGC 2940
   TCCCTCGTGC GCTCTCCTGT TCCGACCCTG CCGCTTACCG GATACCTGTC CGCCTTTCTC 3000
   CCTTCGGGAA GCGTGGCGCT TTCTCATAGC TCACGCTGTA GGTATCTCAG TTCGGTGTAG 3060
   GTCGTTCGCT CCAAGCTGGG CTGTGTGCAC GAACCCCCCG TTCAGCCCGA CCGCTGCGCC 3120
   TTATCCGGTA ACTATCGTCT TGAGTCCAAC CCGGTAAGAC ACGACTTATC GCCACTGGCA 3180
   GCAGCCACTG GTAACAGGAT TAGCAGAGCG AGGTATGTAG GCGGTGCTAC AGAGTTCTTG 3240
   AAGTGGTGGC CTAACTACGG CTACACTAGA AGGACAGTAT TTGGTATCTG CGCTCTGCTG 3300
   AAGCCAGTTA CCTTCGGAAA AAGAGTTGGT AGCTCTTGAT CCGGCAAACA AACCACCGCT 3360
   GGTAGCGGTG GTTTTTTTGT TTGCAAGCAG CAGATTACGC GCAGAAAAAA AGGATCTCAA 3420
   GAAGATCCTT TGATCTTTTC TACGGGGTCT GACGCTCAGT GGAACGAAAA CTCACGTTAA 3480
   GGGATTTTGG TCATGAGATT ATCAAAAAGG ATCTTCACCT AGATCCTTTT AAATTAAAAA 3540
   TGAAGTTTTA AATCAATCTA AAGTATATAT GAGTAAACTT GGTCTGACAG TTACCAATGC 3600
   TTAATCAGTG AGGCACCTAT CTCAGCGATC TGTCTATTTC GTTCATCCAT AGTTGCCTGA 3660
   CTCCCCGTCG TGTAGATAAC TACGATACGG GAGGGCTTAC CATCTGGCCC CAGTGCTGCA 3720
   ATGATACCGC GAGACCCACG CTCACCGGCT CCAGATTTAT CAGCAATAAA CCAGCCAGCC 3780
   GGAAGGGCCG AGCGCAGAAG TGGTCCTGCA ACTTTATCCG CCTCCATCCA GTCTATTAAT 3840
   TGTTGCCGGG AAGCTAGAGT AAGTAGTTCG CCAGTTAATA GTTTGCGCAA CGTTGTTGCC 3900
   ATTGCTACAG GCATCGTGGT GTCACGCTCG TCGTTTGGTA TGGCTTCATT CAGCTCCGGT 3960
   TCCCAACGAT CAAGGCGAGT TACATGATCC CCCATGTTGT GCAAAAAAGC GGTTAGCTCC 4020
   TTCGGTCCTC CGATCGTTGT CAGAAGTAAG TTGGCCGCAG TGTTATCACT CATGGTTATG 4080
   GCAGCACTGC ATAATTCTCT TACTGTCATG CCATCCGTAA GATGCTTTTC TGTGACTGGT 4140
   GAGTACTCAA CCAAGTCATT CTGAGAATAG TGTATGCGGC GACCGAGTTG CTCTTGCCCG 4200
   GCGTCAATAC GGGATAATAC CGCGCCACAT AGCAGAACTT TAAAAGTGCT CATCATTGGA 4260
   AAACGTTCTT CGGGGCGAAA ACTCTCAAGG ATCTTACCGC TGTTGAGATC CAGTTCGATG 4320
   TAACCCACTC GTGCACCCAA CTGATCTTCA GCATCTTTTA CTTTCACCAG CGTTTCTGGG 4380
   TGAGCAAAAA CAGGAAGGCA AAATGCCGCA AAAAAGGGAA TAAGGGCGAC ACGGAAATGT 4440
   TGAATACTCA TACTCTTCCT TTTTCAATAT TATTGAAGCA TTTATCAGGG TTATTGTCTC 4500
   ATGAGCGGAT ACATATTTGA ATGTATTTAG AAAAATAAAC AAATAGGGGT TCCGCGCACA 4560
   TTTCCCCGAA AAGTGCCACC TAA 4583
(2) INFORMATION ZU SEQ ID NO: 20:
   (i) SEQUENZ CHARAKTERISTIKA:
      (A) LÄNGE: 4102 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..4102
   (xi) SEQUENZBESCHREIBUNG: SEQ ID NO: 20:
   ATTGTAAGCG TTAATATTTT GTTAAAATTC GCGTTAAATT TTTGTTAAAT CAGCTCATTT 60
   TTTAACCAAT AGGCCGAAAT CGGCAAAATC CCTTATAAAT CAAAAGAATA GACCGAGATA 120
   GGGTTGAGTG TTGTTCCAGT TTGGAACAAG AGTCCACTAT TAAAGAACGT GGACTCCAAC 180
   GTCAAAGGGC GAAAAACCGT CTATCAGGGC GATGGCCCAC TACGTGAACC ATCACCCTAA 240
   TCAAGTTTTT TGGGGTCGAG GTGCCGTAAA GCACTAAATC GGAACCCTAA AGGGAGCCCC 300
   CGATTTAGAG CTTGACGGGG AAAGCCGGCG AACGTGGCGA GAAAGGAAGG GAAGAAAGCG 360
   AAAGGAGCGG GCGCTAGGGC GCTGGCAAGT GTAGCGGTCA CGCTGCGCGT AACCACCACA 420
   CCCGCCGCGC TTAATGCGCC GCTACAGGGC GCGTCCCATT CGCCATTCAG GCTGCGCAAC 480
   TGTTGGGAAG GGCGATCGGT GCGGGCCTCT TCGCTATTAC GCCAGCTGGC GAAAGGGGGA 540
   TGTGCTGCAA GGCGATTAAG TTGGGTAACG CCAGGGTTTT CCCAGTCACG ACGTTGTAAA 600
   ACGACGGCCA GTGAGCGCGC GTAATACGAC TCACTATAGG GCGAATTGGA GCTCCACCGC 660
   GGTGGCGCTC TAGAACTAGT GGATCCGGCC ATGGAGGCCT TCAAGAATTA GCTTTTCAAT 720
   TCAATTCATC ATTTTTTTTT TATTCTTTTT TTTGATTTCG GTTTCTTTGA AATTTTTTTG 780
   ATTCGGTAAT CTCCGAACAG AAGGAAGAAC GAAGGAAGGA GCACAGACTT AGATTGGTAT 840
   ATATACGCAT ATGTAGTGTT GAAGAAACAT GAAATTGCCC AGTATTCTTA ACCCAACTGC 900
   ACAGAACAAA AACATGCAGG AAACGAAGAT AAATCATGTC GAAAGCTACA TATAAGGAAC 960
   GTGCTGCTAC TCATCCTAGT CCTGTTGCTG CCAAGCTATT TAATATCATG CACGAAAAGC 1020
   AAACAAACTT GTGTGCTTCA TTGGATGTTC GTACCACCAA GGAATTACTG GAGTTAGTTG 1080
   AAGCATTAGG TCCCAAAATT TGTTTACTAA AAACACATGT GGATATCTTG ACTGATTTTT 1140
   CCATGGAGGG CACAGTTAAG CCGCTAAAGG CATTATCCGC CAAGTACAAT TTTTTACTCT 1200
   TCGAAGACAG AAAATTTGCT GACATTGGTA ATACAGTCAA ATTGCAGTAC TCTGCGGGTG 1260
   TATACAGAAT AGCAGAATGG GCAGACATTA CGAATGCACA CGGTGTGGTG GGCCCAGGTA 1320
   TTGTTAGCGG TTTGAAGCAG GCGGCAGAAG AAGTAACAAA GGAACCTAGA GGCCTTTTGA 1380
   TGTTAGCAGA ATTGTCATGC AAGGGCTCCC TATCTACTGG AGAATATACT AAGGGTACTG 1440
   TTGACATTGC GAAGAGCGAC AAAGATTTTG TTATCGGCTT TATTGCTCAA AGAGACATGG 1500
   GTGGAAGAGA TGAAGGTTAC GATTGGTTGA TTATGACACC CGGTGTGGGT TTAGATGACA 1560
   AGGGAGACGC ATTGGGTCAA CAGTATAGAA CCGTGGATGA TGTGGTCTCT ACAGGATCTG 1620
   ACATTATTAT TGTTGGAAGA GGACTATTTG CAAAGGGAAG GGATGCTAAG GTAGAGGGTG 1680
   AACGTTACAG AAAAGCAGGC TGGGAAGCAT ATTTGAGAAG ATGCGGCCAG CAAAACTAAA 1740
   AAACTGTATT ATAAGTAAAT GCATGTATAC TAAACTCACA AATTAGAGCT TCAATTTAAT 1800
   TATATCAGTT ATTACCCAAT TCTCATGTTT GCGGCCGCTG CAGGAATTCG ATATCAAGCT 1860
   TATCGATACC GTCGACCTCG AGGGGGGGCC CGGTACCCAG CTTTTGTTCC CTTTAGTGAG 1920
   GGTTAATTGC GCGCTTGGCG TAATCATGGT CATAGCTGTT TCCTGTGTGA AATTGTTATC 1980
   CGCTCACAAT TCCACACAAC ATACGAGCCG GAAGCATAAA GTGTAAAGCC TGGGGTGCCT 2040
   AATGAGTGAG CTAACTCACA TTAATTGCGT TGCGCTCACT GCCCGCTTTC CAGTCGGGAA 2100
   ACCTGTCGTG CCAGCTGCAT TAATGAATCG GCCAACGCGC GGGGAGAGGC GGTTTGCGTA 2160
   TTGGGCGCTC TTCCGCTTCC TCGCTCACTG ACTCGCTGCG CTCGGTCGTT CGGCTGCGGC 2220
   GAGCGGTATC AGCTCACTCA AAGGCGGTAA TACGGTTATC CACAGAATCA GGGGATAACG 2280
   CAGGAAAGAA CATGTGAGCA AAAGGCCAGC AAAAGGCCAG GAACCGTAAA AAGGCCGCGT 2340
   TGCTGGCGTT TTTCCATAGG CTCCGCCCCC CTGACGAGCA TCACAAAAAT CGACGCTCAA 2400
   GTCAGAGGTG GCGAAACCCG ACAGGACTAT AAAGATACCA GGCGTTTCCC CCTGGAAGCT 2460
   CCCTCGTGCG CTCTCCTGTT CCGACCCTGC CGCTTACCGG ATACCTGTCC GCCTTTCTCC 2520
   CTTCGGGAAG CGTGGCGCTT TCTCATAGCT CACGCTGTAG GTATCTCAGT TCGGTGTAGG 2580
   TCGTTCGCTC CAAGCTGGGC TGTGTGCACG AACCCCCCGT TCAGCCCGAC CGCTGCGCCT 2640
   TATCCGGTAA CTATCGTCTT GAGTCCAACC CGGTAAGACA CGACTTATCG CCACTGGCAG 2700
   CAGCCACTGG TAACAGGATT AGCAGAGCGA GGTATGTAGG CGGTGCTACA GAGTTCTTGA 2760
   AGTGGTGGCC TAACTACGGC TACACTAGAA GGACAGTATT TGGTATCTGC GCTCTGCTGA 2820
   AGCCAGTTAC CTTCGGAAAA AGAGTTGGTA GCTCTTGATC CGGCAAACAA ACCACCGCTG 2880
   GTAGCGGTGG TTTTTTTGTT TGCAAGCAGC AGATTACGCG CAGAAAAAAA GGATCTCAAG 2940
   AAGATCCTTT GATCTTTTCT ACGGGGTCTG ACGCTCAGTG GAACGAAAAC TCACGTTAAG     3000
   GGATTTTGGT CATGAGATTA TCAAAAAGGA TCTTCACCTA GATCCTTTTA AATTAAAAAT     3060
   GAAGTTTTAA ATCAATCTAA AGTATATATG AGTAAACTTG GTCTGACAGT TACCAATGCT     3120
   TAATCAGTGA GGCACCTATC TCAGCGATCT GTCTATTTCG TTCATCCATA GTTGCCTGAC     3180
   TCCCCGTCGT GTAGATAACT ACGATACGGG AGGGCTTACC ATCTGGCCCC AGTGCTGCAA     3240
   TGATACCGCG AGACCCACGC TCACCGGCTC CAGATTTATC AGCAATAAAC CAGCCAGCCG     3300
   GAAGGGCCGA GCGCAGAAGT GGTCCTGCAA CTTTATCCGC CTCCATCCAG TCTATTAATT     3360
   GTTGCCGGGA AGCTAGAGTA AGTAGTTCGC CAGTTAATAG TTTGCGCAAC GTTGTTGCCA     3420
   TTGCTACAGG CATCGTGGTG TCACGCTCGT CGTTTGGTAT GGCTTCATTC AGCTCCGGTT     3480
   CCCAACGATC AAGGCGAGTT ACATGATCCC CCATGTTGTG CAAAAAAGCG GTTAGCTCCT     3540
   TCGGTCCTCC GATCGTTGTC AGAAGTAAGT TGGCCGCAGT GTTATCACTC ATGGTTATGG     3600
   CAGCACTGCA TAATTCTCTT ACTGTCATGC CATCCGTAAG ATGCTTTTCT GTGACTGGTG     3660
   AGTACTCAAC CAAGTCATTC TGAGAATAGT GTATGCGGCG ACCGAGTTGC TCTTGCCCGG     3720
   CGTCAATACG GGATAATACC GCGCCACATA GCAGAACTTT AAAAGTGCTC ATCATTGGAA     3780
   AACGTTCTTC GGGGCGAAAA CTCTCAAGGA TCTTACCGCT GTTGAGATCC AGTTCGATGT     3840
   AACCCACTCG TGCACCCAAC TGATCTTCAG CATCTTTTAC TTTCACCAGC GTTTCTGGGT     3900
   GAGCAAAAAC AGGAAGGCAA AATGCCGCAA AAAAGGGAAT AAGGGCGACA CGGAAATGTT     3960
   GAATACTCAT ACTCTTCCTT TTTCAATATT ATTGAAGCAT TTATCAGGGT TATTGTCTCA     4020
   TGAGCGGATA CATATTTGAA TGTATTTAGA AAAATAAACA AATAGGGGTT CCGCGCACAT     4080
   TTCCCCGAAA AGTGCCACCT AA                                              4102
(2) INFORMATION ZU SEQ ID NO: 21:
   (i) SEQUENZ CHARAKTERISTIKA:
      (A) LÄNGE: 3956 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..3956
   (xi) SEQUENZBESCHREIBUNG: SEQ ID NO: 21:
   ATTGTAAGCG TTAATATTTT GTTAAAATTC GCGTTAAATT TTTGTTAAAT CAGCTCATTT     60
   TTTAACCAAT AGGCCGAAAT CGGCAAAATC CCTTATAAAT CAAAAGAATA GACCGAGATA    120
   GGGTTGAGTG TTGTTCCAGT TTGGAACAAG AGTCCACTAT TAAAGAACGT GGACTCCAAC    180
   GTCAAAGGGC GAAAAACCGT CTATCAGGGC GATGGCCCAC TACGTGAACC ATCACCCTAA    240
   TCAAGTTTTT TGGGGTCGAG GTGCCGTAAA GCACTAAATC GGAACCCTAA AGGGAGCCCC    300
   CGATTTAGAG CTTGACGGGG AAAGCCGGCG AACGTGGCGA GAAAGGAAGG GAAGAAAGCG    360
   AAAGGAGCGG GCGCTAGGGC GCTGGCAAGT GTAGCGGTCA CGCTGCGCGT AACCACCACA    420
   CCCGCCGCGC TTAATGCGCC GCTACAGGGC GCGTCCCATT CGCCATTCAG GCTGCGCAAC    480
   TGTTGGGAAG GGCGATCGGT GCGGGCCTCT TCGCTATTAC GCCAGCTGGC GAAAGGGGGA    540
   TGTGCTGCAA GGCGATTAAG TTGGGTAACG CCAGGGTTTT CCCAGTCACG ACGTTGTAAA    600
   ACGACGGCCA GTGAGCGCGC GTAATACGAC TCACTATAGG GCGAATTGGA GCTCCACCGC    660
   GGTGGCGCTC TAGAACTAGT GGATCCGGCC ATGGAGGCCA CACCGCATAG ATCGGCAAGT    720
   GCACAAACAA TACTTAAATA AATACTACTC AGTAATAACC TATTTCTTAG CATTTTTGAC    780
   GAAATTTGCT ATTTTGTTAG AGTCTTTTAC ACCATTTGTC TCCACACCTC CGCTTACATC    840
   AACACCAATA ACGCCATTTA ATCTAAGCGC ATCACCAACA TTTTCTGGCG TCAGTCCACC    900
   AGCTAACATA AAATGTAAGC TTTCGGGGCT CTCTTGCCTT CCAACCCAGT CAGAAATCGA    960
   GTTCCAATCC AAAAGTTCAC CTGTCCCACC TGCTTCTGAA TCAAACAAGG GAATAAACGA   1020
   ATGAGGTTTC TGTGAAGCTG CACTGAGTAG TATGTTGCAG TCTTTTGGAA ATACGAGTCT   1080
   TTTAATAACT GGCAAACCGA GGAACTCTTG GTATTCTTGC CACGACTCAT CTCCATGCAG   1140
   TTGGACGATA TCAATGCCGT AATCATTGAC CAGAGCCAAA ACATCCTCCT TAGGTTGATT   1200
   ACGAAACACG CCAACCAAGT ATTTCGGAGT GCCTGAACTA TTTTTATATG CTTTTACAAG   1260
   ACTTGAAATT TTCCTTGCAA TAACCGGGTC AATTGTTCTC TTTCTATTGG GCACACATAT   1320
   AATACCCAGC AAGTCAGCAT CGGAATCTAG AGCACATTCT GCGGCCTCTG TGCTCTGCAA 1380
   GCCGCAAACT TTCACCAATG GACCAGAACT ACCTGTGAAA TTAATAACAG ACATACTCCA 1440
   AGCTGCCTTT GTGTGCTTAA TCACGTATAC TCACGTGCTC AATAGTCACC AATGCCCTCC 1500
   CTCTTGGCCC TCTCCTTTTC TTTTTTCGAC CGAATTAATT CTTAATCGGC AAAAAAAGAA 1560
   AAGCTCCGGA TCAAGATTGT ACGTAAGGTG ACAAGCTATT TTTCAATAAA GAATATCTTC 1620
   CACTACTGCC ATCTGGCGTC ATAACTGCAA AGTACACATA TATTACGATG CTGTTCTATT 1680
   AAATGCGGCC GCTGCAGGAA TTCGATATCA AGCTTATCGA TACCGTCGAC CTCGAGGGGG 1740
   GGCCCGGTAC CCAGCTTTTG TTCCCTTTAG TGAGGGTTAA TTGCGCGCTT GGCGTAATCA 1800
   TGGTCATAGC TGTTTCCTGT GTGAAATTGT TATCCGCTCA CAATTCCACA CAACATACGA 1860
   GCCGGAAGCA TAAAGTGTAA AGCCTGGGGT GCCTAATGAG TGAGCTAACT CACATTAATT 1920
   GCGTTGCGCT CACTGCCCGC TTTCCAGTCG GGAAACCTGT CGTGCCAGCT GCATTAATGA 1980
   ATCGGCCAAC GCGCGGGGAG AGGCGGTTTG CGTATTGGGC GCTCTTCCGC TTCCTCGCTC 2040
   ACTGACTCGC TGCGCTCGGT CGTTCGGCTG CGGCGAGCGG TATCAGCTCA CTCAAAGGCG 2100
   GTAATACGGT TATCCACAGA ATCAGGGGAT AACGCAGGAA AGAACATGTG AGCAAAAGGC 2160
   CAGCAAAAGG CCAGGAACCG TAAAAAGGCC GCGTTGCTGG CGTTTTTCCA TAGGCTCCGC 2220
   CCCCCTGACG AGCATCACAA AAATCGACGC TCAAGTCAGA GGTGGCGAAA CCCGACAGGA 2280
   CTATAAAGAT ACCAGGCGTT TCCCCCTGGA AGCTCCCTCG TGCGCTCTCC TGTTCCGACC 2340
   CTGCCGCTTA CCGGATACCT GTCCGCCTTT CTCCCTTCGG GAAGCGTGGC GCTTTCTCAT 2400
   AGCTCACGCT GTAGGTATCT CAGTTCGGTG TAGGTCGTTC GCTCCAAGCT GGGCTGTGTG 2460
   CACGAACCCC CCGTTCAGCC CGACCGCTGC GCCTTATCCG GTAACTATCG TCTTGAGTCC 2520
   AACCCGGTAA GACACGACTT ATCGCCACTG GCAGCAGCCA CTGGTAACAG GATTAGCAGA 2580
   GCGAGGTATG TAGGCGGTGC TACAGAGTTC TTGAAGTGGT GGCCTAACTA CGGCTACACT 2640
   AGAAGGACAG TATTTGGTAT CTGCGCTCTG CTGAAGCCAG TTACCTTCGG AAAAAGAGTT 2700
   GGTAGCTCTT GATCCGGCAA ACAAACCACC GCTGGTAGCG GTGGTTTTTT TGTTTGCAAG 2760
   CAGCAGATTA CGCGCAGAAA AAAAGGATCT CAAGAAGATC CTTTGATCTT TTCTACGGGG 2820
   TCTGACGCTC AGTGGAACGA AAACTCACGT TAAGGGATTT TGGTCATGAG ATTATCAAAA 2880
   AGGATCTTCA CCTAGATCCT TTTAAATTAA AAATGAAGTT TTAAATCAAT CTAAAGTATA 2940
   TATGAGTAAA CTTGGTCTGA CAGTTACCAA TGCTTAATCA GTGAGGCACC TATCTCAGCG 3000
   ATCTGTCTAT TTCGTTCATC CATAGTTGCC TGACTCCCCG TCGTGTAGAT AACTACGATA 3060
   CGGGAGGGCT TACCATCTGG CCCCAGTGCT GCAATGATAC CGCGAGACCC ACGCTCACCG 3120
   GCTCCAGATT TATCAGCAAT AAACCAGCCA GCCGGAAGGG CCGAGCGCAG AAGTGGTCCT 3180
   GCAACTTTAT CCGCCTCCAT CCAGTCTATT AATTGTTGCC GGGAAGCTAG AGTAAGTAGT 3240
   TCGCCAGTTA ATAGTTTGCG CAACGTTGTT GCCATTGCTA CAGGCATCGT GGTGTCACGC 3300
   TCGTCGTTTG GTATGGCTTC ATTCAGCTCC GGTTCCCAAC GATCAAGGCG AGTTACATGA 3360
   TCCCCCATGT TGTGCAAAAA AGCGGTTAGC TCCTTCGGTC CTCCGATCGT TGTCAGAAGT 3420
   AAGTTGGCCG CAGTGTTATC ACTCATGGTT ATGGCAGCAC TGCATAATTC TCTTACTGTC 3480
   ATGCCATCCG TAAGATGCTT TTCTGTGACT GGTGAGTACT CAACCAAGTC ATTCTGAGAA 3540
   TAGTGTATGC GGCGACCGAG TTGCTCTTGC CCGGCGTCAA TACGGGATAA TACCGCGCCA 3600
   CATAGCAGAA CTTTAAAAGT GCTCATCATT GGAAAACGTT CTTCGGGGCG AAAACTCTCA 3660
   AGGATCTTAC CGCTGTTGAG ATCCAGTTCG ATGTAACCCA CTCGTGCACC CAACTGATCT 3720
   TCAGCATCTT TTACTTTCAC CAGCGTTTCT GGGTGAGCAA AAACAGGAAG GCAAAATGCC 3780
   GCAAAAAAGG GAATAAGGGC GACACGGAAA TGTTGAATAC TCATACTCTT CCTTTTTCAA 3840
   TATTATTGAA GCATTTATCA GGGTTATTGT CTCATGAGCG GATACATATT TGAATGTATT 3900
   TAGAAAAATA AACAAATAGG GGTTCCGCGC ACATTTCCCC GAAAAGTGCC ACCTAA 3956
(2) INFORMATION ZU SEQ ID NO: 22:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..59
   (xi) SEQUENZBESCHREIBUNG: SEQ ID NO: 22:
      ATAGGCGCTT CTCGTATCTA TACTCAACCC GCCCCCAATG CAGCTGAAGC TTCGTACGC 59
(2) INFORMATION ZU SEQ ID NO: 23:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 23:
   AAATTGGGGG CACAAATGAG GGGTAAAAAT GCAGACATTA GCATAGGCCA CTAGTGGATC 60
   TG 62
(2) INFORMATION ZU SEQ ID NO: 24:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..59
   (xi) SEQUENZBESCHREIBUNG: SEQ ID NO: 24:
      TCTAAATCGT TATGTTGAAA ACCTAGGCAC CAATGTGACT CAGCTGAAGC TTCGTACGC 59
(2) INFORMATION ZU SEQ ID NO: 25:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 25:
   CAGCTTTTGC CCAATATGCT CAAAACCGAG TTATCTATTA GCATAGGCCA CTAGTGGATC 60
   TG 62
(2) INFORMATION ZU SEQ ID NO: 26:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..59
   (xi) SEQUENZBESCHREIBUNG: SEQ ID NO: 26:
      CAAGTTACTT TGAAAGGAAA TAAAAAAAAT TGTCAGCATG CAGCTGAAGC TTCGTACGC 59
(2) INFORMATION ZU SEQ ID NO: 27:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 27:
   ATATTTGATG CAATTTCTGC CTTAAAGTAC AAAATGCTTA GCATAGGCCA CTAGTGGATC 60
   TG 62
(2) INFORMATION ZU SEQ ID NO: 28:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..59
   (xi) SEQUENZBESCHREIBUNG: SEQ ID NO: 28:
      AATATTCATA AAACAGGATC TTTCAAGGGA CGATAAAATG CAGCTGAAGC TTCGTACGC 59
(2) INFORMATION ZU SEQ ID NO: 29:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 27:
(2) INFORMATION ZU SEQ ID NO: 30:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomiach)
   (ix) MERKMALE:
      (A) NAME/SCHLÜSSEL: exon
      (B) LAGE: 1..59
   (xi) SEQUENZBESCHREIBUNG: SEQ ID NO: 30:
      TCAATCGAAG CATTTGAAGC ATACTCTAGA CCAAAGAAGA CAGCTGAAGC TTCGTACGC 59
(2) INFORMATION ZU SEQ ID NO: 31:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 31:
   TTGAATTCAA GACAAAAAAT CAAATCTTGC TGAGTTGTTA GCATAGGCCA CTAGTGGATC 60
   TG 62
(2) INFORMATION ZU SEQ ID NO: 32:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..59
   (xi) SEQUENZBESCHREIBUNG: SEQ ID NO: 32:
      GAAGCCTGGC TATACCAATC CGGCTTTAAA AGCCCTTGGT CAGCTGAAGC TTCGTACGC 59
(2) INFORMATION ZU SEQ ID NO: 33:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 33:
   CTTTACCCTG TTTGACCCAG TTCTGTGGCC AATCTTTTTC GCATAGGCCA CTAGTGGATC 60
   TG 62
(2) INFORMATION ZU SEQ ID NO: 34:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..59
   (xi) SEQUENZBESCHREIBUNG: SEQ ID NO: 34:
      TTCCTAAAAG TAATTCTTAA AAGTGATAAT GAATGACTTA CAGCTGAAGC TTCGTACGC 59
(2) INFORMATION ZU SEQ ID NO: 35:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 35:
   ACCTAGTTGA AAAGATTTGT TCCGCAGATA AGAAAAAATG GCATAGGCCA CTAGTGGATC 60
   TG 62
(2) INFORMATION ZU SEQ ID NO: 36:
   (i) SEQUENZ CHARAKTERISTIKA:
      (A) LÄNGE: 59 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..59
   (xi) SEQUENZBESCHREIBUNG: SEQ ID NO: 36:
      CACAGGGCCG CATTATTTCT TTGATTTCGT TTTTTTCACC CAGCTGAAGC TTCGTACGC 59
(2) INFORMATION ZU SEQ ID NO: 37:
   (i) SEQUENZ CHARAKTERISTIKA:
      (A) LÄNGE: 62 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..62
   (xi) SEQUENZBESCHREIBUNG: SEQ ID NO: 37:
   GATTTAGAGA TTCAAACTCC GTTATTTTTA GAAGGTCATG GCATAGGCCA CTAGTGGATC 60
   TG 62

## Beschreibung

Die Erfindung betrifft ein Verfahren zum Screening von antimykotisch wirksamen Substanzen, wobei ein essentielles Gen aus Saccharomyces als Target eingesetzt wird.

Das Spektrum der bekannten Pilzinfektionen reicht von Pilzbefällen an der Hautoberfläche oder der Nägel bis hin zu potentiell lebenbedrohlichen mykotischen Infektionen der inneren Organe. Derartige Infektionen und damit einhergehende Folgeerkrankungen, werden als Mykosen bezeichnet.

Zur Behandlung von Mykosen werden antimykotisch (fungistatisch oder fungizid) wirkende Substanzen eingesetzt. Bisher gibt es aber nur relativ wenige pharmakologisch wirksame Substanzen, wie beispielsweise Amphotericin B, Nystatin, Pimaricin, Griseofulvin, Clotrimazol, 5-Fluorcytosin und Batraphen. Die medikamentiöse Behandlung von Pilzinfektionen ist außerordentlich schwierig, insbesondere deshalb, weil es sich sowohl bei den Myceten als auch bei den Wirtszellen um eukaryotische Zellen handelt. Die Einnahme von Arzneimitteln, die die bekannten antimykotischen Wirkstoffe enthalten ist deshalb oft mit unerwünschten Nebenwirkungen verbunden, beispielsweise wirkt Amphotericin B nephrotoxisch. Es besteht also ein starker Bedarf an pharmakologisch wirksamen Substanzen, die zur Herstellung von Medikamenten eingesetzt werden können, die bei einer Schwächung des Immunsystems prophylaktisch oder im Falle einer bereits vorliegenden Infektion zur Behandlung von Mykosen eingesetzt werden können. Dabei sollten die Substanzen ein spezifisches Wirkprofil aufweisen, so daß selektiv das Wachstum und die Vermehrung von Myceten verhindert werden kann, ohne dabei den Wirtsorganismus zu schädigen.

Für die Identifizierung antimykotisch wirksamer Substanzen fehlt es bisher an kompatiblen, aussagekräftigen Testverfahren.

In WO 95/11969 wird ein Verfahren zum Screening von antimykotischen Substanzen beschrieben, wobei der Effekt von zu testenden Substanzen auf die Translation eines Proteins als Maß für die Wirkung der zu testenden Substanz bestimmt wird.

Eine Aufgabe der vorliegenden Erfindung ist es, ein Verfahren zur Identifizierung von antimykotisch wirkenden Substanzen zu entwickeln, welches möglichst universell einsetzbar ist und die Testung einer Vielzahl von potentiellen Wirkstoffen in einer möglichst effektiven Weise ermöglicht.

Ein wesentliches Merkmal des Verfahrens ist, daß ein essentielles Gen aus Saccharomyces cerevisiae als Target für das Screening verwendet wird. Dieses Verfahren unterscheidet sich insbeondere dadurch von bekannten Verfahren, daß keine detaillierten Kenntnisse über die biochemische Funktion des durch das essentielle Gen kodierten Proteins notwendig sind.

Die Erfindung betrifft ein Verfahren zum Auffinden von antimykotisch wirkenden Substanzen, wobei mindestens eine Saccharomyces cerevisiae Zelle bereitgestellt wird, die das Gen YGR046w aufgrund der Transformation mit einem rekombinanten Expressionsvektor, der den kodierenden Teil des YGR046w Gens unter der Kontrolle eines regulierbaren Promotors enthält gegenüber Vergleichszellen überexprimiert, diese Zelle mit einer zu untersuchenden Substanz inkubiert wird und die wachstumsinhibierende Wirkung dieser Substanz bestimmt wird. Insbesondere werden antimykotisch wirkende Substanzen dadurch aufgefunden, daß sie die funktionelle Expression des essentiellen Gens (Transkription und Translation) aus Saccharomyces cerevisiae die funktionelle Aktivität des kodierten Proteins ganz oder teilweise inhibieren.

Der Erfindung liegt zugrunde, daß zuerst essentielle Gene In Saccharomyces cerevisiae identifiziert wurden.

Um essentielle Gene in S. cerevisiae zu identifizieren, werden einzelne Gene von S. cerevisiae durch homologe Rekombination aus dem Genom der S. cerevisiae entfernt. Handelt es sich bei dem entfernten DNA-Abschnitt um ein essentielles Gen, dann ist die Deletion letal für die S. cerevisiae Zellen.

Um entsprechende Deletionen im Genom von S. cerevisiae zu erzeugen und solche S. cerevisiae Zellen, die die Deletion tragen selektionieren zu können, wird eine Methode verwendet, bei dem das zu untersuchende S. cerevisiae Gen durch ein Markergen ersetzt wird. Über dieses Markergen (Gen eines Selektionsmarkers) können die Zellen selektioniert werden, bei denen eine homologe Rekombination stattgefunden hat, da bei ihnen das zu untersuchende Gen durch das Gen eines Selektionsmarkers ersetzt worden ist. Als Selektionsmarker können z. B. dominante Selektionsmarker oder Auxotrophiemarker verwendet werden.

Als Auxotrophiemarker werden Gene benutzt, die für Schlüsselenzyme der Aminosäure- oder Nukleobasen-Synthesewege kodieren. Beispielsweise können S. cerevisiae Gene die für Enzyme aus dem Aminosäurestoffwechsel von Leucin (z. B. LEU2-Gen), Histidin (z. B. HIS3-Gen) oder Tryptophan (z. B. TRP1-Gen) oder aus dem der Stoffwechsel der Nukleobase Uracil (z. B. URA3-Gen) kodieren, als Marker verwendet werden.

Das Verfahren beinhaltet, daß auxotrophe S. cerevisiae Zellen bzw. Stämme verwendet werden können, d. h. Zellen bzw. Stämme, in denen das für den jeweils verwendeten Marker kodierende Gen ein oder mehrere Mutationen aufweist, wodurch gewährleistet wird, daß kein funktionell aktives Enzym exprimiert wird. Diese auxotrophen Zellen bzw. Stämme können nur in Nährmedien wachsen, in denen die entsprechenden Aminosäuren oder Nukleobasen enthalten sind. Als Stämme können beispielsweise alle S. cerevisiae Laborstämme verwendet werden, die Auxotrophie- und/oder Nukleobasen-Marker besitzen. Werden diploide S. cerevisiae Zellen bzw. Stämme eingesetzt dann, müssen die entsprechenden Markergene homozygot mutiert vorliegen. Insbesondere wird der Stamm CEN.PK2 (Scientific Research & Development GmbH, Oberursel, Deutschland) oder isogene Derivate dieses Stamms eingesetzt.

Das Verfahren beinhaltet auch, daß S. cerevisiae Zellen bzw. Stämme verwendet werden, die keine geeigneten Auxotrophiemarker besitzen beispielsweise prototrophe S. cerevisiae Zellen bzw. Stämme. Dann können dominante Selektionsmarker, beispielsweise Resistenzgene, wie das Kanamycin-Resistenzgen als Marker eingesetzt werden.

Für die homologe Rekombination, bei der in Genen von S. cerevisiae die DNA-Sequenz des zu untersuchenden S. cerevisiae Gens ganz oder teilweise durch die Sequenz des Markergens ersetzt wird, werden DNA-Fragmente eingesetzt, bei denen das Markergen am 5'- und am 3'-Ende von Sequenzen flankiert wird, die zu Sequenz-Abschnitten am 5'- und 3'-Ende des zu untersuchenden S. cerevisiae Gens homolog sind.

Für die Herstellung entsprechender DNA-Fragmente sind verschiedene Verfahren möglich, die zur Deletion spezifischer S. cerevisiae Gene etwa gleich gut geeignet sind. Für die Transformation in eine geeignete S. cerevisiae Zelle bzw. einen Stamm wird ein lineares DNA-Fragment eingesetzt. Dieses wird durch die homologe Rekombination ins S. cerevisiae Genom integriert.

In dem Verfahren können drei verschiedene Methoden verwendet werden:
1. "Klassische Methode" zur Erzeugung von Deletionskassetten (Rothstein, R. J. (1983) Methods in Enzymology Vol. 101, 202 - 211).
2. "Klassische Methode" unter Verwendung der PCR-Technik ("Modifizierte Klassische Methode").
3. SFH (short flanking homology)-PCR-Methode (Wach, A. et al. (1994) Yeast 10: 1793 - 1808; Güldner, U. et al. (1996) Nucleic Acids Research 24: 2519 - 2524).

1. Bei der "Klassischen Methode" zur Erzeugung von Deletionskassetten im S. cerevisiae Genom liegt das zu untersuchende Gen entweder bereits in einem geeigneten Vektor vor oder es wird in einen solchen integriert. Bei dieser Methode können beispielsweise alle pBR-, pUC- und pBluescript®-Derivate verwendet werden. Aus diesen Vektoren wird, z. B. unter Verwendung geschickt ausgewählter Restriktionsschnittstellen, ein Großteil der Sequenz des zu untersuchenden Gens entfernt, wobei aber die 5'- und 3'-Bereiche des zu untersuchenden Gens im Vektor verbleiben. Zwischen diese verbleibenden Bereiche wird das Gen des ausgewählten Selektionsmarkers integriert.
2. Bei einer modifizierten Form dieser "Klassischen Methode" wird die PCR-Technik eingesetzt. Bei dieser Methode werden die Bereiche des zu untersuchenden S. cerevisiae Gens, die sich am 3'- beziehungsweise 5'-Ende der kodierenden Sequenz befinden mit Hilfe der PCR-Technik amplifiziert. Bei diesem Verfahren werden ausschließlich die Randbereiche beidseitig des zu untersuchenden Gens amplifiziert, weshalb für jedes zu untersuchende Gen zwei PCR-Reaktionen durchgeführt werden müssen, wobei einmal das 5'- und einmal das 3'-Ende des Gens amplifiziert wird. Die Länge der amplifizierten DNA-Abschnitte der Randbereiche hängt beispielsweise von den in diesen Bereichen vorhandenen Restriktionsschnittstellen ab. Die amplifizierten Randbereiche des zu untersuchenden Gens haben in der Regel eine Länge von 50 bis 5000 Basenpaaren, besonders bevorzugt eine Länge zwischen 500 und 1000 Basenpaaren (Bp).

Als Template für die PCR-Reaktion kann beispielsweise genomische DNA aus S. cerevisiae eingesetzt werden. Als Template für die PCR-Reaktionen können Wildtyp-Gene oder modifizierte Wildtyp-Gene verwendet werden. Die Primer-Paare (jeweils ein Sense- und ein Antisense-Primer) werden so konstruiert, daß sie Sequenz-Abschnitten am 3'- bzw. 5'-Ende des zu untersuchenden S. cerevisiae Gens entsprechen. Insbesondere werden die Primer so gewählt, daß eine Integration über geeignete Restriktionsschnittstellen in den Vektor möglich ist.

Als Vektoren können Derivate des pUC-, pBR- und pBluescript®-Vektors eingesetzt werden. Insbesondere sind Vektoren geeignet, die bereits ein Gen, das für einen Selektionsmarker kodiert, enthalten. Insbesondere können hierzu Vektoren verwendet werden, die die Gene der Selektionsmarker HIS3, LEU2, TRP1 oder URA3 enthalten. Beispielsweise können hierzu die Plasmide pPK5/6 (SEQ ID NO. 18), pPK7/8 (SEQ ID NO. 19), pPK9/10 (SEQ ID. NO. 20) und pPK13/14 (SEQ ID NO. 21) verwendet werden. Die Nukleotidsequenz der Plasmide pPK5/6, pPK7/8, pPK9/10 und pPK13/14 ist im Sequenzprotokoll angegeben. Die Herstellung dieser Plasmide ist in den Beispielen 2 bis 6 beschrieben.

Die mittels PCR erzeugten DNA-Abschnitte des zu untersuchenden S. cerevisiae Gens werden beiderseits des im Vektor bereits vorhandenen Gens, das für den Selektionsmarker kodiert, in den Vektor integriert, so daß anschließend, wie bei der "Klassischen Methode", der verwendete Selektionsmarker beidseitig von homologen DNA-Sequenzen des zu untersuchenden Gens flankiert vorliegt.
3. Da überraschenderweise die homolge Rekombination an S. cerevisiae sehr effizient und präzise verläuft, kann die Länge der zu dem zu untersuchenden S. cerevisiae Gen homologen DNA-Abschnitte, die das Gen des Selektionsmarkers flankieren, gegebenenfalls deutlich kürzer gewählt werden als bei der "Modifizierten Klassischen Methode". Die flankierenden, zu dem zu untersuchenden Gen homologen Bereiche brauchen nur eine Länge von etwa 20 - 60 Basenpaaren, besonders bevorzugt von 30 - 45 Basenpaaren, aufzuweisen. Besonders vorteilhaft an der SFH-PCR-Methode ist, daß aufwendige Klonierungsschritte entfallen.

An einem DNA-Template, daß das Gen für den zu verwendenden Selektionsmarker enthält, wird eine PCR-Reaktion durchgeführt, wobei die verwendeten Primer so konstruiert werden, daß die DNA-Sequenz des Sense-Primers dem 5'- Ende der Sequenz des Selektionsmarkers homolog ist und daß der Primer zusätzlich an seinem 5'-Ende einen vorzugsweise 40 Nukleotide langen Bereich aufweist, der der Sequenz am 5'-Ende des zu untersuchenden S. cerevisiae Gens entspricht. Analog wird der Antisense-Primer so konstruiert, daß er zu dem 3'-Ende der Sequenz des Gens des Selektionsmarkers komplementär ist, wobei dieser Primer an seinem 5'-Ende einen vorzugsweise ebenfalls 40 Nukleotide langen Bereich enthält, der der Sequenz am 3'-Ende des zu untersuchenden Gens entspricht.

Für die Amplifikation von zu untersuchenden S. cerevisiae Genen mit Hilfe der SFH-PCR-Methode werden beispielsweise Vektoren verwendet, die bereits das Gen für einen Auxotrophie- oder einen Selektionsmarker enthalten. Insbesondere wird das Plasmid pUG6 als Template verwendet. Dieses Plasmid enthält eine loxP-KanMX-loxP Kassette (Güldener, U. et al. (1996) Nucleic Acids Research 24: 2519 - 2524). Das heißt, ein Kanamycin-Resistenzgen ist beidseitig von einer loxP-Sequenz (loxP-KanMX-loxP Kassette) flankiert. Die Verwendung dieser Kassette hat den Vorteil, daß nach der Integration der loxP-KanMX-loxP Kassette an den Genort an dem das zu untersuchende S. cerevisiae Gen lokalisiert war, das Kanamycin-Resistenzgen gegebenenfalls wieder aus dem S. cerevisiae Genom entfernt werden kann. Dafür kann die Cre-Rekombinase des Bakteriophagen P1 verwendet werden.

Die Cre-Rekombinase erkennt die IoxP-Sequenzen und entfernt die zwischen den beiden IoxP-Sequenzen liegende DNA durch einen homologen Rekombinationsprozeß. Als Ergebnis bleibt nur eine IoxP-Sequenz zurück und es kommt zur sogenannten Marker-Rückgewinnung, d. h. der S. cerevisiae Stamm kann erneut mit IoxP-KanMX-IoxP Kassette transformiert werden. Dies ist besonders vorteilhaft, wenn zwei oder mehrere funktionshomologe Gene deletieren werden sollen, um einen letalen Phänotyp zu erhalten.

Bei der SFH-PCR-Methode werden in der PCR-Reaktion Primer verwendet, die an ihrem 3'-Ende einen vorzugsweise etwa 20 Nukleotide langen Bereich aufweisen, der zu Sequenzen links beziehungsweise rechts von der loxP-KanMX-loxP Kassette homolog ist, wobei die Primer jeweils an ihrem 5'-Ende einen vorzugsweise 40 Nukleotide langen Bereich aufweisen, der Sequenzabschnitten an den Enden des zu untersuchenden Gens homolog ist.

Mit allen drei Methoden werden lineare Deletionskassetten erhalten, die das Gen für einen ausgewählten Selektionsmarker enthalten, der beidseitig von homologen Sequenzen des zu untersuchenden Gens flankiert wird. Diese Deletionskassetten werden für die Transformation diploider S. cerevisiae Stämme eingesetzt. Beispielsweise kann hierfür der diploide S. cerevisiae Stamm CEN.PK2 (Scientific Research & Development GmbH, Oberursel, Deutschland) verwendet werden.

### CEN.PK2 Mata/MAT a ura3-52/ura3-52 leu2-3, 112/leu2-3, 112 his3Δ1/his3Δ1 trp1-289/trp1-289 MAL2-8^{c}/MAL2-8^{c} SUC2/SUC2

Der Stamm CEN.PK2 wird nach bekannten Verfahren angezogen und kultiviert (Gietz, R. D. et al. (1992) Nucleic Acids Research 8: 1425; Güldener, U. et al. (1996) Nucleic Acids Research 24: 2519-2524).

Die Zellen des verwendeten S. cerevisiae Stammes werden mit einer entsprechenden Menge DNA der linearen Deletionskassette nach bekannten Verfahren transformiert (beispielsweise Sambrook et al. (1989) Molecular Cloning, A Laboratory Manual. Cold Spring Habor Laboratory Press). Danach wird das Medium in dem die Zellen kultiviert werden gegen neues Medium, sogenanntes Selektivmedium, das die entsprechende Aminosäure (beispielsweise Histidin, Leucin oder Tryptophan) oder Nukleobase (beispielsweise Uracil) nicht enthält bzw. bei Verwendung einer Deletionskassette die das Kanamycin-Resistenzgen enthält, in Medien mit Geniticin (G418®) kultiviert. Alternativ können die transformierten Zellen auf mit entsprechendem Medium hergestellten Agarplatten ausplattiert werden. Auf diese Weise werden die Transformanden selektioniert, bei denen eine homologe Rekombination stattgefunden hat, da nur diese Zellen unter den veränderten Bedingungen wachsen können.

In den meisten Fällen wird bei der Transformation einer diploiden S. cerevisiae Zelle bzw. eines Stammes aber nur eine der beiden in dem doppelten Chromosomensatz vorliegenden Kopien des zu untersuchenden Gens durch die DNA der Deletions-Kassette ersetzt, so daß eine heterozygot-diploide S. cerevisiae Zelle bzw. ein heterozygot-diploider S. cerevisiae Mutantenstamm entsteht, bei der/dem eine Kopie des zu untersuchenden Gens durch das Gen des Selektionsmarkers ersetzt wird, während die andere Kopie des zu untersuchenden Gens im Genom erhalten bleibt. Das hat den Vorteil, daß falls auf diese Weise ein essentielles Gen deletiert wird, die heterozygot-diploide Zelle bzw. der S. cerevisiae Mutantenstamm aufgrund der noch vorhandenen zweiten Kopie des essentiellen Gens weiterhin lebensfähig ist.

Die korrekte Integration der DNA der Deletionskassette an den vorbestimmen chromosomalen Genlocus (Genlocus des zu untersuchenden Gens) kann gegebenenfalls mit Hilfe der Southern-Blot-Analyse (Southern, E. M. (1975) J. Mol. Biol. 98: 503 - 517) oder mit Hilfe der diagnostischen PCR-Analyse unter Verwendung spezifischer Primer überprüft werden (Güldener, U. et al. (1996) Nucleic Acids Research 24: 2519 - 2524).

Die genetische Aufspaltung einzelner diploider Zellen läßt sich mit einer Tetradenanalyse verfolgen. Dazu werden diploide Stämme, insbesondere heterozygot-diploide Mutantenstämme, mit Hilfe von bekannten Verfahren zur Reduktionsteilung (Meiose) angeregt, beispielsweise durch Stickstoffverarmung auf Kaliumacetat-Platten (Sherman, F. et al. (1986) Cold Spring Harbor Laboratory Press, Cold Spring Harbor, N. Y.; Guthrie, C. und Fink, G. R. (1991) Methods in Enzymology. Volume 194. Academic Press, San Diego, 3 - 21; Ausubel, F. M. et al. (1987) Current Protocols in Molecular Biology, John Wiley & Sons, Inc., Chapter 13). Die Meiose resultiert in Asci mit vier Ascosporen (Segreganten), die nach enzymatischen Partialverdauung der Ascussporenwand mit Zymolyase (Ausubel et al. (1987)) mit Hilfe eines Mikromanipulators (z.B. Firma SINGER) vereinzelt werden können. Wird beispielsweise ein heterozygot-diploider Mutantenstamm, bei dem ein essentielles Gen im doppelten Chromosomensatz durch die homologe Rekombination ausgetauscht wurde einer Tetraden-Analyse unterzogen, dann überleben nur zwei Segreganten, nämlich die, die das essentielle Gen noch tragen. Die beiden anderen Segreganten sind nicht lebensfähig, weil bei ihnen das zu untersuchende, in diesem Fall essentielle Gen, fehlt.

Zur Verifikation, ob die auf diese Weise untersuchten Gene tatsächlich essentiell sind oder ob dem Genlocus des zu untersuchenden Gens benachbarte, möglicherweise essentielle Gene durch die homologe Rekombination "beschädigt" wurden, werden die heterozygot-diploiden S. cerevisiae Mutantenstämme mit einem Centromerplasmid, in dem das zu untersuchende Gen vorliegt, transformiert. Die Transformanden werden einer Tetraden-Analyse unterzogen. Werden dann wieder vier anstelle von zwei lebensfähigen Segreganten erhalten, so kann das im Centromerplasmid vorliegende zu untersuchende Gen den Defekt der zwei nicht lebensfähigen haploiden S. cerevisiae Zellen/Mutantenstämme komplementieren, womit bewiesen ist, daß das untersuchte S. cerevisiae Gen essentiell ist.

Vorzugsweise werden als Centromerplasmide Plasmide verwendet, die in niedriger Kopienzahl, beispielsweise in 1 oder 2 Kopien pro Zelle vorliegen. Beispielsweise können hierzu die Plasmide pRS313, pRS314, pRS315 und pRS316 (Sikorski, R. S. und Hieter, P. (1989) Genetics 122: 19 - 27) oder ähnliche Plasmide verwendet werden. Dabei werden die zu untersuchenden Gene vorzugsweise einschließlich ihrer 5'- und 3'-nichtkodierenden Bereiche in diese Plasmide integriert.

Mit den beschriebenen Methoden lassen sich einzelne Gene von Saccharomyces cerivisae, deren DNA-Sequenz ganz oder teilweise bekannt ist, untersuchen. Die komplette DNA-Sequenz des S. cerevisiae Genoms wurde am 24. April 1996 über das World Wide Wep (WWW) veröffentlicht.

Folgende Möglichkeiten bestehen, um auf DNA-Sequenzen des S. cerevisiae Genoms übers WWW zuzugreifen.
MIPS (Munich Information Centre of Protein Sequence)
   Adresse http://speedy.mips.biochem.mpg.de/mips/yeast/yeast-genom.htmlx
SGD (Saccharomyces Genome Database, Stanford)
   Adresse http://genome-www.stanford.edu/Saccharomyces
YPD (Yeast Protein Database, Cold Spring Harbor)
   Adresse: http://www.prteome.com/YPDhome.html

Die vollständige DNA-Sequenz des S. Cerevisiae Genoms ist auch über FTP (file transfer protocol) in Europa (z.B. unter der Adresse: ftp.mips.embnet.org) in den USA (Adresse: genome-ftp.stanford.edu) oder in Japan (Adresse: ftp.nig.ac.jp) zugänglich.

Unter Verwendung der beschriebenen Methoden kann mit Hilfe dieser Sequenzinformation für jedes einzelne Saccharomyces cerevisiae Gen bestimmt werden, ob das jeweilige Gen für S. cerevisiae essentiell ist.

Im Genom von S. cerevisiae wurden auf diese Weise die Gene YGR046w, YGR048w, YGR060w, YJL074c, YJR136c, YJR141w, YBR167c, YPL252c, YPL242c, YOR119c, YPL235w, YOR110w, YNL182c, YOR206w, YJL054w, YJL039c, YNL258c, YNL245c, YNL038w, YNL251c, YNL256w, YNL260c, YIR012w, YLR086w, YLR076c, YLR100w, YIR010w, YIL003w, YBR102c, YOL010w, YKL013c, YKL018w und YLL003w als essentiell identifiziert.

Tabelle 6 gibt einen Überblick über diese essentiellen Gene und die damit in Zusammenhang stehenden Informationen. In Spalte 1 sind die Bezeichnungen für die erzeugten Mutantenstamm (CEN.PK2 Stämme, in denen das essentielle Gen durch ein Markergen ausgetauscht wurde), in Spalte 2 die systematischen Gen-Namen der essentiellen Gene (Bezeichnung unter der die entsprechenden DNA-Sequenzen in Datenbanken geführt werden), in Spalte 3 die zur Herstellung des Mutantenstämme verwendeten Selektionsmarker sowie in den Spalten 4 und 5 die deletierten Nukleotide und die dazu korrespondierenden Aminosäuren der essentiellen Gene (als Bezugspunkt dient die Position 1; Position 1 ist das A des voraussichtlichen Start-Codons ATG des offenen Leserahmens) angegeben. Soweit verfügbar, sind auch die Gen-Namen (Spalte 6) und Einträge in Datenbanken DB (Spalte 7), angegeben. Insbesondere sind dort Eintragungen aus Datenbanken über die Essentialität der Gene vermerkt. Beispielsweise bei dem Gen YGR060w ist vermerkt, daß dieses Gen zuvor als nicht essentiell klassifiziert wurde. Unter Verwendung des CEN.PK2 Stammes wurde nun überraschenderweise gefunden, daß das YGR060w Gen doch essentiell ist. Darüber hinaus sind in Spalte 8, soweit vorhanden, weitere Informationen z.B. bzgl. der Funktion der als essentiell identifizierten Gene oder der kodierten Proteine und/oder Homologien/Ähnlichkeiten zu anderen Genen oder Proteinen angegeben.

Die Angaben in Tabelle 6 unterstreichen, daß obwohl die DNA-Sequenzen der S. cerivisae Gene (Spalte 2) bekannt sind, über die Funktion, oder die charakteristischen Eigenschaften dieser Gene bzw. der kodierten Proteine bisher kaum etwas bekannt ist und auch die essentielle Funktion dieser Gene oder der durch diese Gene kodierten Proteine bisher nicht bekannt war.

Unter dem systematischen Gennamen (Spalte 2 in Tabelle 6) sind die Sequenzen der als essentiell identifizierten Gene in Gendatenbanken, z. B. den o.g. zugänglich. Die Erfindung betrifft die Verwendung der essentiellen Gene YGR046w, YGR048w, YGR060w, YJL074c, YJR136c, YJR141w, YBR167c, YPL252c, YPL242c, YOR119c, YPL235w, YOR110w, YNL182c, YOR206w, YJL054w, YJL039c, YNL258c, YNL245c, YNL038w, YNL251c, YNL256w, YNL260c, YIR012w, YLR086w, YLR076c, YLR100w, YIR010w, YIL003w, YBR102c, YOL010w, YKL013c, YKL018w und YLL003w.

Ausgehend von dem Stamm CEN.PK2 (Scientific Research & Technologie GmbH, Oberursel, Deutschland) wurden unter Verwendung einer der drei genannten Methoden die in Tabelle 6, Spalte 1 genannten Saccharomyces cerevisiae Stämme CEN.EN27, CEN.EN28, CEN.EN8, CEN.RO23, CEN.R030, CEN.RO6, CEN.RO8, CEN.SR14, CEN.SR15, CEN.SR2, CEN.SR26, CEN.SR41, CEN.SR55, CEN.SR66, CEN.SR80, CEN.SR81, CEN.HE1, CEN.HE17, CEN.HE18, CEN.HE2, CEN.HE4, CEN.HE9, CEN.HI10, CEN.HI23, CEN.H128, CEN.H131, CEN.H15, CEN.HI7, CEN.FE8, CEN.KR28, CEN.TS02, CEN.TS04 und CEN.ZI26 erzeugt. Diese Stämme sind dadurch definiert, daß die in Tabelle 6, Spalte 4 angegebenen Nukleotide (bzw. die in Spalte 5 angegebenen Aminosäuren) durch die in Tabelle 6, Spalte 3 angegeben Selektionsmarker ersetzt wurden.

Das Verfahren beinhaltet, daß das essentielle Gen YGR046W aus Saccharomyces cerevisiae eingesetzt wird, um Substanzen zu identifizieren, die die funktionelle Expression dieses essentiellen Gens aus S. cerevisiae und/oder die funktionelle Aktivität des kodierten Proteins ganz oder teilweise inhibieren können. Mit Hilfe dieses Verfahren können Substanzen identifiziert werden, die das Wachstum von Myceten inhibieren und die als Antimykotika, beispielsweise zur Herstellung von Arzneimitteln, eingesetzt werden können.

Ein besonderes Merkmal des Verfahrens ist, daß für das Screening der Substanzen ein essentielles Gen aus Saccharomyces cerevisiae als Target eingesetzt wird.

Das Verfahren beinhaltet, daß Zellen, mindestens eine S. cerevisiae Zelle, die das essentielle Gen, das als Target eingesetzt wird, überexprimiert, bereitgestellt wird und daß diese Zelle mit einer zu testenden Substanz inkubiert wird. Auf diese Weise kann die wachstumsinhibierende Wirkung dieser Substanz im Bezug auf das essentielle Target-Gen bestimmt werden. Das Gen, welches in diesem Verfahren untersucht wird, wird auch als Target-Gen bzw. zu untersuchendes Gen bezeichnet. Das erfindungsgemäße Target Gen ist das Gen YGR046w.

In dem Verfahren wird der wachstumsinhibierende Effekt einer Substanz auf eine Zelle, in der das Target-Gen überexprimiert wird, bestimmt. Dabei kann die Substanz entweder die Expression des essentiellen Gens und/oder die funktionette Aktivität des kodierten Proteins inhibieren.

Eine weitere Ausführungsform ist, daß Saccharomyces cerevisiae Zellen, die das Target-Gen in unterschiedlichem Maße exprimieren, bereitgestellt werden und daß diese Zellen dann mit einer zu testenden Substanz inkubiert werden und die wachstumsinhibierende Wirkung dieser Substanz auf die Zellen vergleichend bestimmt wird.

Das Verfahren beinhaltet, daß zwei oder mehrere Saccharomyces cerevisiae Zellen die sich dadurch unterscheiden, daß sie das Target-Gen in unterschiedlichem Maße exprimieren, verwendet werden. Beispielsweise können zwei, drei, vier, fünf, zehn oder mehr Zellen bzw. die dazu korrespondierenden Stämme im Bezug auf die wachstumsinhibierende Wirkung einer Substanz, die in einer definierten Konzentration eingesetzt wird, vergleichend analysiert werden. Durch solche Konzentrationsreihen können beispielsweise antimykotisch wirkende Substanzen von cytotoxischen oder nicht wirksamen Substanzen unterschieden werden.

Eine besondere Ausführungsform des Verfahrens ist, daß für das Screening haploide S. cerevisiae Zellen/Stämme verwendet werden.

Das Verfahren beinhaltet, daß das als Target ausgewählte essentielle Gen in einen geeigneten Expressionsvektor integriert wird.

Als Expressionsvektoren eignen sich beispielsweise E. coli/S. cerevisiae Pendel-Vektoren. Insbesondere können Vektoren eingesetzt werden, die sich in ihrer Kopienzahl pro Zelle unterscheiden. Beispielsweise können einerseits Vektoren verwendet werden, die in hoher Kopien-Zahl in transformierten S. cerevisiae Zellen vorliegen als auch solche Vektoren, die in niedriger Kopienzahl vorliegen. Eine Ausführungsform ist, daß Expressionsvektoren eingesetzt werden, die eine Integration des Target-Gens ins S. cerevisiae Genom erlauben.

Als Expressionsvektoren eignen sich beispielsweise die Vektoren pRS423, pRS424, pRS425, pRS426, pRS313, pRS314, pRS315, pRS316, pRS303, pRS304, pRS305, pRS306 (Sikorski und Hieter, 1989; Christianson, et al., 1992).

Die Vektoren der Serie pRS423-pRS426 liegen in hoher Kopienzahl (etwa 50 - 100 Kopien/Zelle) vor. Im Gegensatz dazu liegen die Vektoren der Serie pRS313-pRS316 in niedriger Kopienzahl (1-2 Kopien/Zellen) vor. Werden Expressionsvektoren aus diesen beiden Serien verwendet, dann liegt das Target-Gen als extrachromosomale Kopie vor. Mit Hilfe der Vektoren der Serie pRS303-pRS306 können die Target-Gene ins Genom integriert werden. Durch die Verwendung dieser drei verschiedenen Expressionsvektor-Typen, die sich nur bezüglich ihrer in S. cerevisiae Zellen vorliegenden Kopienzahl unterscheiden, ist eine differenzierte bzw. abgestufte Expression des essentiellen S. cerevisiae Gens bzw. des funktionsähnlichen Gens möglich.

Das Verfahren beinhaltet, daß vergleichend die wachstumsinhibierende Wirkung von Substanzen im Bezug auf die Zellen/Stämme, die mit unterschiedlichen Expressionsvektoren, die sich z. B. in der Kopienzahl des Vektors/Zelle unterscheiden, bestimmt wird. Solche Zellen können das essentielle Target-Gen in unterschiedlichem Maße exprimieren und eine abgestufte Reaktion auf die Substanz zeigen.

Das Verfahren beinhaltet auch, daß eine unterschiedlich starke Expression des Target-Gens in verschiedenen Zellen (geregelte Überexpression) dadurch erreicht wird, daß das Target-Gen in Expressionsvektoren zwischen speziell ausgewählte Promotoren und Terminatoren, beispielsweise S. cerevisiae Promotoren und Terminatoren inseriert wird.

Für die geregelte Überexpression des Target-Gens eignen sich regulierbare Promotoren. Beispielsweise können die nativen Promotoren der Gene GAL1 bzw. entsprechende Derivate der Promotoren, z. B. solche, bei denen verschiedene UAS-Elemente entfernt wurden (GALS, GALL; Mumberg et al., (1994) Nucleic Acids Research 22: 5767 - 5768) sowie Promotoren gluconeogenetischer Gene, z. B. die Promotoren FBP1, PCK1, ICL1 oder Teile dieser Promotoren, z. B. deren Aktivator-(UAS1 bzw. UAS2) oder Repressor- (URS) Sequenzen in entsprechenden nicht aktivierbaren bzw. reprimierbaren Test-Promotoren (Schüller et al., (1992) EMBO J. 11: 107 - 114) Guarente et al., (1984) Cell 36: 503 - 511; Niederacher et al. (1992) Curr. Genet. 22: 363 - 370; Proft et al. (1995) Mol. Gen. Genet. 246: 367 - 373;) eingesetzt werden.

In den Expressionsvektoren können als Terminatoren beispielsweise die Terminatorsequenzen der S. cerevisiae Gene MET25, PGK1, TPI1, TDH3, ADHI, URA3 verwendet werden.

Das Verfahren beinhaltet, daß durch die Verwendung, geschickt ausgewählter Expressionsvektor-Typen und/oder die Herstellung von geeigneten Expressionsvektoren, gegebenenfalls unter Benutzung verschiedenen starker Promotoren und/oder unterschiedlich regulierter Promotoren, eine Reihe von Expressionsvektoren hergestellt werden können, die alle das gleiche Target-Gen enthalten, sich aber dadurch unterscheiden, daß sie das Target-Gen in unterschiedlichem Maße (unterschiedlich stark) exprimieren. Mit Hilfe solcher Reihen von Expressionsvektoren ist es möglich, eine in ihrer Stärke fein abgestufte Expression des Target-Gens zu erzielen. Mit Hilfe solcher Reihen von Expressionsvektoren ist es möglich, S. cerevisiae Zellen/Stämme herzustellen, die das Target-Gen in unterschiedlichem Maße exprimieren.

Das Verfahren beinhaltet, daß die Expressionsvektoren in haploide Wildtyp-Zellen von S. cerevisiae transformiert werden. Die so erhaltenen Zellen/Stämme werden in Flüssigmedium angezogen, mit verschiedenen Konzentrationen der zu untersuchenden Substanz inkubiert und die Wirkung dieser Substanz auf das Wachstumsverhalten der Zellen/Stämme vergleichend analysiert, die das Target-Gen in unterschiedlichem Maße exprimieren. Das Verfahren beinhaltet auch, daß als Referenz haploide S. cerevisiae Zellen/Stämme, die mit dem jeweiligen Expressionsvektor-Typ ohne Target-Gen transformiert wurden, verwendet werden.

Das Verfahren beinhaltet, daß bei der Verwendung von regulierbaren Promotoren, insbesondere beim Einsatz des GAL1 Promotors und dessen Derivaten (GALS und GALL) das Screening von Substanzen in verschiedenen Medien durchgeführt werden kann, da hier die Expressionsstärke durch die Wahl des jeweiligen Mediums stark zu beeinflussen ist. So nimmt die Expressionsstärke des GAL1 Promotors in folgender Weise ab: 2 % Galaktose > 1 % Galaktose + 1 % Glukose > 2 % Glycerin > 2 % Glukose.

Die wachstumsinhibierende Wirkung von Substanzen, die Wldtyp-Zellen von S. cerevisiae in ihrem Wachstum inhibieren, kann durch die Überexpression des essentiellen S. cerevisiae Gens ganz oder teilweise aufgehoben werden.

Ein besonderer Vorteil des Screeningverfahrens ist, daß es ausreicht zu wissen, daß das verwendete Gen essentiell ist; über die Funktion des essentiellen Gens oder die Funktion des kodierten Proteins ist keine weitere Informationen notwendig.

Besondere Vorteile des Verfahrens sind:
- Es ist keinerlei Kenntnis Ober die biochemische Funktion des essentiellen Gens aus S. cerevisiae notwendig.
- In dem Verfahren zum Auffinden antimykotisch wirksamer Substanzen wird nicht differenziert, ob die Substanz die funktionelle Expression des essentiellen Gens inhibiert oder ob sie die funktionelle Aktivität des kodierten Proteins inhibiert.
- Einzelne Substanzen können auf diese Weise effizient auf ihre spezifische Wtrksamkeit getestet werden.

### Beispiele:

### Beispiel 1: Die "Klassische Methode" zur Erzeugung von Deletions-Kassetten dargestellt für den YJR141w (Tabelle 6).

Deletion des ORF YJR141w-Gens aus S. cerevisiae unter Verwendung des HIS3-Gens von S. cerevisiae:
1) Klonierung eines 1,7kB Xbal-Fragments (enthalten entweder aus genornische S. cerevisiae DNA bzw. aus einem entsprechenden Cosmid-Klon, der das YJR141w-Gen beinhaltet) in einem puC18 Vektor, der mit dem Restriktionsenzym Xbal linearisiert wurde.
2) Das aus 1.) erhaltene Plasmid wurde zunächst mit dem Restriktionsenzym BstEII geschnitten und nach dem Auffüllen der überstehenden DNA-Enden mit Hilfe des Enzyms Klenow-Polymerase (Sambrook et al., 1989) das erhaltene lineare DNA-Fragment mit dem Restriktionsenzym Clal geschnitten. Dadurch wurde ein 3,52kB und ein 0,87kB (Kilobasenpaare) großes DNA-Fragment erhalten.
3) Das HIS3-Gen von S. cerevisiae wird als genomisches 1,6kB BamHI-Fragment in den mit dem Restriktionsenzym BamHI geschnittenen Vektor pBluescript IIKS+ (Stratagene) inseriert und auf diese Weise das Plasmid pMR240 hergestellt.
4) Das Plasmid pMR240 wurde zunächst mit dem Restriktionsenzym Xhol geschnitten und dann die überstehenden DNA-Enden mit Hilfe der Klenow-Polymerase aufgefüllt. Das lineare DNA-Fragment wurde mit dem Restriktionsenzym Clal geschnitten. Dadurch wurde ein 1,36kB DNA-Fragment, daß das HIS3-Gen von S. cerevisiae enthält, erhalten.
5) Das 3,52kB DNA-Fragment aus 2.) wurde mit dem aus 4.) erhaltenen 1,36kB DNA-Fragment ligiert und auf diese Weise das Plasmid pR06 erzeugt. Im Plasmid pR06 wurde ein 870Bp langer DNA-Abschnitt des kodierenden Bereichs von YJR 141w deletiert und gegen den Selektionsmarker HIS3 ersetzt.
6) Das Plasmid pR06 wurde mit der Restriktionsendonuklease Pvull linearisiert und zur Transformation von S. cerevisiae eingesetzt.

### Beispiel 2: Konstruktion von Plasmiden für die SFH-Methode.

1) Als Ausgangsvektor wurde der Vektor pBluescript®II KS+ (Stratagene; Sequenz verfügbar: Genbank® X52327) verwendet.
2) Der Vektor pBluescript®II KS+ wurde mit dem Restriktionsenzym NotI linearisiert und die überstehenden Enden durch anschließende Inkubation mit Mungbohnen 5'-3'Exonuklease entfernt. Durch Religation des verkürzten DNA-Fragments wurde der Vektor pKS+ΔNotl hergestellt (Vektor pBluescript®II KS ohne die Notl Restriktionsschnittstelle).
3) Das Plasmid pKS+ΔNotI wurde mit den Restriktionsenzymen PstI und BamHI geschnitten und das DNA-Oligonuleotid, welches aus dem Primer-Paar PK3/PK4 erzeugt wurde, in das geöffnete Plasmid ligiert. Das auf diese Weise erzeugte Plasmid pKS+neu (SEQ ID. NO. 17) enthält zwischen der Pstl und BamHI Restriktionsschnittstelle die neuen Restriktionsschnittstellen Notl, Stul, Sfil und Ncol:
   PstI-NotI-StuI-SfiI-NcoI-BamHI
      SEQ ID NO. 3: 5'-GCGGCCGCAAGGCCTCCATGGCCG-3' PK3
      SEQ ID NO. 4: 5'-GATCCGGCCATGGAGGCCTTGCGGCCGCTGCA-3' PK4
4) Das Plasmid pKS+neu (SEQ ID. NO. 17) diente als Ausgangsvektor zur Herstellung der Plasmide pPK5/6 (SEQ ID NO. 18), pPK7/8 (SEQ ID NO. 19), pPK9/10 (SEO ID NO. 20) und pPK13/14 (SEQ ID NO. 21). Die Gene für die entsprechenden Aminosäure-/Nukleobasen-Auxotrophie-Marker wurden mittels PCR unter Verwendung geeigneter Primer an den Wildtyp- bzw. modifizierten Wildtyp-Genen (Beispiel 4 und Beispiel 5) amplifiziert.

### Beispiel 3: Konstruktion des Plasmids pPK5/6 (pKS+neu - HIS 3) (SEQ ID NO. 18):

Unter Verwendung der Primer PK5 und PK6 wurde mittels PCR an genomischer S. cerevisiae DNA das HIS3-Gen amplifiziert, anschließend mit den Restriktionsenzymen BamHI und Notl geschnitten und in das mit BamHI und NotI geschnittene Plasmid pKS+neu inseriert. Die unterstrichenen DNA-Abschnitte der Primer entsprechen den Abschnitten, die der jeweiligen homologen Sequenzen der S. cerevisiae Gene entsprechen.

### Beispiel 4: Konstruktion des Plasmids pK7/8 (pKS+neu - LEU2) (SEQ ID NO. 19):

Unter Verwendung der Primer PK7 und PK8 wurde mittels PCR an Ycplac111 Vektor-DNA (Gietz, R. D. und Sugino, A. (1988) Gene 74: 527 - 534) als Matrize (modifiziertes Wildtyp-Gen) das LEU2 Gen von S. cerevisiae amplifiziert, die amplifizierte DNA anschließend mit den Restriktionsenzymen BamHI und NotI geschnitten und in ein mit BamHI und NotI geschnittenes Plasmid pKS+neu inseriert.

### Beispiel 5: Konstruktion des Plasmids pPK9/10 (pKS+neu - URA3) (SEQ ID NO. 20):

Unter Verwendung der Primer PK9 und PK10 wurde mittels PCR an Ycplac33 Vektor-DNA (Gietz, R. D. und Sugino, A. (1988) Gene 74: 527 - 534) als Matrize (modifiziertes Wildtyp-Gen) das URA3 Gen von S. cerevisiae amplifiziert, die amplifizierte DNA anschließend mit den Restriktionsenzymen BamHI und NotI geschnitten und in das mit BamHI und Notl geschnittene Plasmid pKS+neu inseriert.

### Beispiel 6: Konstruktion des Plasmids pPK13/14 (pKS+neu - TRP1) (SEQ ID NO. 21):

Unter Verwendung der Primer PK13 und PK14 wurde mittels PCR an genomischer S. cerevisiae DNA das TRP1-Gen amplifiziert, die amplifizierte DNA anschließend mit den Restriktionsenzymen BamHI und Pstl geschnitten und in das mit BamHI und Pstl geschnittene Plasmid pKS+neu inseriert.

### Beispiel 7: "Klassische Methode" unter Verwendung der PCR-Technik ("Modifizierte klassische Methode") dargestellt für den YGR046w.

1) Unter Verwendung der Primer XSL-2N2 und G385-1 (vergleiche Punkt 4.) wurde der 3'-Bereich des YGR046w an genomischer S. cerevisiae DNA als Matrize amplifiziert. Die amplifizierte DNA wurde anschließend mit den Restriktionsenzymen Clal und EcoRI geschnitten und das erhaltene 508Bp DNA-Fragment in das zuvor mit den Restriktionsenzymen Clal und EcoRl geschnittene Plasmid pPK9/10 ligiert. Das resultierende Plasmid wurde mit p119-58 bezeichnet.
2) Unter Verwendung der Primer G385-3 und X9R2 (vergleiche Punkt 4.) wurde der 5'-Bereich des YGR046w an genomischer S. cerevisiae DNA als Matrize amplifiziert. Die amplifizierte DNA wurde anschließend mit den Restriktionsenzymen BamHI und BglII geschnitten und das erhaltene 1336Bp Fragment in das Plasmid p119-58, das zuvor mit BamHI geschnitten worden war, inseriert. Das resultierende Plasmid wurde mit pEN27 bezeichnet.
3) Das Plasmid pEN27 wurde nach der Linearisierung mit den Restriktionsenzymen SacI und Asp7 18 zur Transformation von S. cerevisiae eingesetzt.
4) Verwendete Primer:
   SEQ ID No. 13:
      XSL-2N2 5'- AGG CAG ACT ACA ACT TAG G -3'
   SEQ ID NO. 14:
      G385-1 5'- CTG AAT TCG ATG AGG AGA AGC TAG T -3'
   SEQ ID NO. 15:
      X9R2 5'- CTT CAA ACG CTT GTT AAA TCT TG -3'
   SEQ ID NO. 16:
      G385-3 5'- CAG GAT CCG TAG ACC ATT TTC AGA A -3'

### Beispiel 8:

S. cerevisiae Zellen vom Stamm CEN.PK2 wurden mit jeweils ca. 5 µg DNA einer linearen Deletionskassette nach bekannten Verfahren transformiert (Gietz et al., 1992; Güldener et al., 1996). Der Transformationsansatz wurde auf entsprechenden Selektivmedien ausplattiert.

Bei Verwendung einer Deletionskassette die das Kanamycinresistenzgen enthielt,wurde auf Vollmedium (YEPD) mit Geniticin (G418®) ausplattiert. Bei der Verwendung einer Deletionskassette die sogenannte Auxotrophie-Marker enthielt wurde auf synthetischen Minimalmedien (SCD), die die entsprechende Aminosäure (Histidin, Leucin oder Tryptophan) bzw. Nukleobase (Uracil) nicht enthielten, ausplattiert. Auf diese Weise wurde auf die Transformanden selektioniert, bei denen eine homologe Rekombination stattgefunden hatte, da nur diese Zellen unter den veränderten Bedingungen wachsen konnten.

**Tabelle 6**

| Name des erzeugten Stammes | systematischer Gen-Name | Selektionsmarker | deletierte Nukleotide | deletierte Aminosäuren | Name des Gens | DB Eintrag | Bemerkungen |
|---|---|---|---|---|---|---|---|
| CEN.EN27 | YGR046w | URA3 | 18-1143 | 9-381 | ∼ | | |
| CEN.EN28 | YGR048w | LEU2 | 73-1077 | 25-359 | UFD1 | YPD kein Eintrag | ubiquitin fusion degradation protein |
| CEN.EN8 | YGR060w | HIS3 | (-)231-836 | (-)77-279 | ERG25 | viable, temperatursensitiv | C-4 sterol methy oxidase |
| CEN.RO23 | YJL074c | HIS3 | 169-3114 | 56-1038 | ∼ | | similarity to Emericella nidulans chromosome scaffold protein |
| CEN.R030 | YJR136c | loxP-KanMX-loxP | 4-1236 | 2-421 | | | |
| CEN.R06 | YJR141w | HIS3 | 27-891 | 10-297 | / | | |
| CEN.RO8 | YBR167c | HIS3 | 110-330 | 37-110 | / | | |
| CEN.SR14 | YPL252c | loxP-KanMX-loxP | 4-516 | 2-172 | | | similarity to adrenoxin and ferrrodoxin |
| CEN.SR15 | YPL242c | IoxP-KanMX-IoxP | 91-4485 | 31-1495 | | | |
| CEN.SR2 | YOR119c | IoxP-KanMX-IoxP | 4-1452 | 2-484 | RIO1 | YPD kein Eintrag | similarity to C. elegans ZK632.3 protein; function unknown |
| CEN.SR26 | YPL235w | IoxP-KanMX-IoxP | 4-1413 | 2-471 | | | homology to hypothetical protein YDR190c |
| CEN.SR41 | YOR110w | LEU2 | 61-1197 | 21-399 | | | homology to hypothetical protein YNL108c |
| CEN.SR55 | YNL182c | loxP-KanMX-loxP | 4-1665 | 2-555 | | | |
| CEN.SR66 | YOR206w | loxP-KanMX-loxP | 73-2130 | 25-710 | | | homology to RAD4 (Frameshift) |
| CEN.SR80 | YJL054w | loxP-KanMX-loxP | 82-1352 | 28-450 | | | |
| CEN.SR81 | YJL039c | IoxP-KanMX-IoxP | 4-5049 | 2-1683 | | | similarity to HSP70 family |
| CEN.HE1 | YNL258c | TRP1 | | | | | |
| CEN.HE17 | YNL245c | loxP-KanMX-loxP | | | | | |
| CEN.HE18 | YNL038w | loxP-KanMX-loxP | | | | | probable membrane protein |
| CEN.HE2 | YNL251 c | loxP-KanMX-loxP | | | NRD1 | | plays a role in sequence specific regulation of nuclear pre m-RNA abundancer |
| CEN.HE4 | YNL256w | loxP-KanMX-loxP | | | | | similarity to bacterial dihydropteroate synthas |
| CEN.HE9 | YNL260c | loxP-KanMX-loxP | | | | | |
| CEN.HI10 | YIR012w | URA3 | | | | | beta transducin repeats |
| CEN.HI23 | YLR086w | loxP-KanMX-IoxP | | | | | similarity to S. pombe cut3 protein |
| CEN.HI28 | YLR076c | IoxP-KanMX-IoxP | | | | | |
| CEN.HI31 | YLR100w | IoxP-KanMX-IoxP | | | | | |
| CEN.HI5 | YIR010w | URA3 | | | | | EF-hand calcium binding domain |
| CEN.HI7 | YIL003w | URA3 | | | | | similarity to E. coli MRP protein; ATPase |
| CEN.FE8 | YBR102c | URA3 | | | | | hypothetical membrane protein |
| CEN.KR28 | YOL010w | IoxP-KanMX-IoxP | | | | | homology to S. pombe SPAC12g12.06c protein |
| CEN.TS02 | YKL013c | IoxP-KanMX-IoxP | | | | | strong similarity to unknown C. elegans protein |
| CEN.TS04 | YKL018w | IoxP-KanMX-IoxP | | | | | |
| CEN.ZI26 | YLL003w | LEU2 | | | "SFI1" | | protein of unknown funktion |

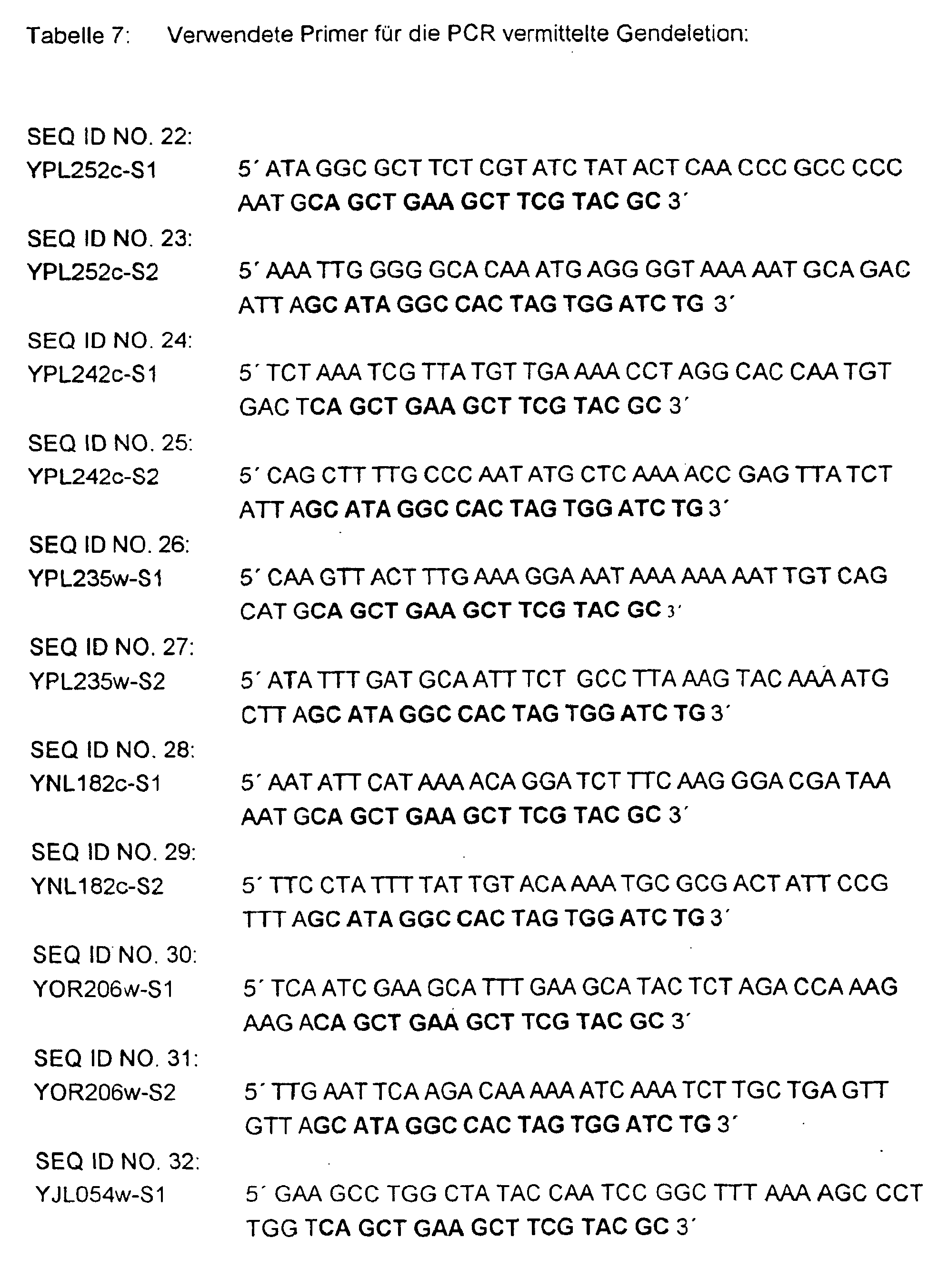

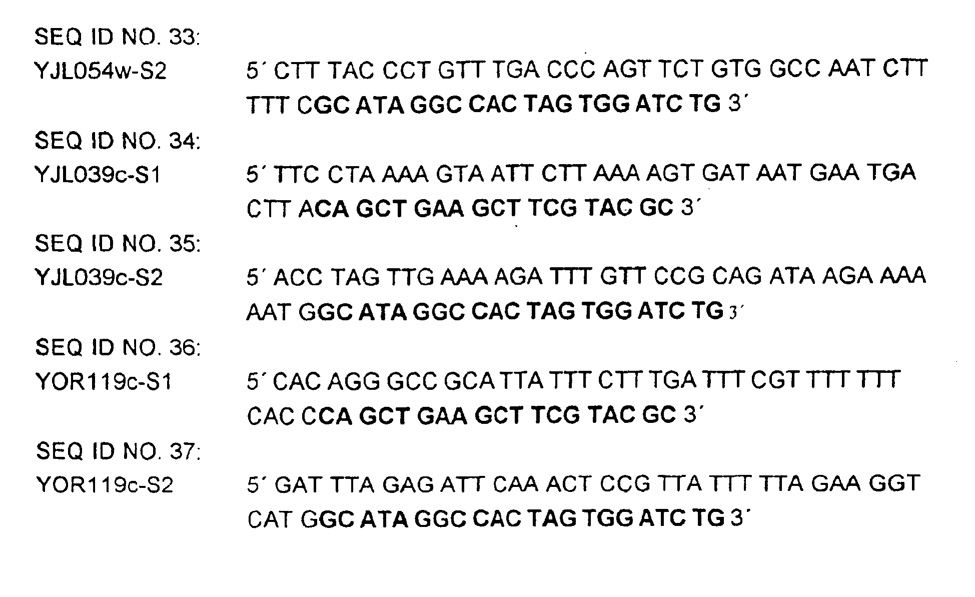

## Patentansprüche

1. Verfahren zum Auffinden von antimykotisch wirkenden Substanzen, **dadurch gekennzeichnet, dass** mindestens eine Saccharomyces cerevisiae Zelle bereitgestellt wird, die das Gen YGR046w aufgrund der Transformation mit einem rekombinanten Expressionsvektor, der den kodierenden Teil des YGR046w Gens unter der Kontrolle eines regulierbaren Promotors enthält gegenüber Vergleichszellen überexprimiert, diese Zelle mit einer zu untersuchenden Substanz inkubiert wird und die wachstumsinhibierende Wirkung dieser Substanz bestimmt wird.

2. Verfahren nach Anspruch 1, wobei mindestens zwei Saccharomyces cerevisiae Zellen bereitgestellt werden, die das Gen YGR046w in unterschiedlichem Maße exprimieren, diese Zellen mit einer zu untersuchenden Substanz inkubiert werden und die wachstumsinhibierende Wirkung dieser Substanz vergleichend bestimmt wird.

3. Verfahren nach einem der Ansprüche 1 bis 2, wobei die Zellen Saccharomyces cerevisiae Zellen des Stammes CEN.PK2 oder Derivate desselben sind.

4. Verfahren nach einem der Ansprüche 1 bis 3, wobei die Zellen haploide Saccharomyces cerevisiae Zellen sind.

5. Verfahren nach einem der Ansprüche 3 oder 4, wobei die Saccharomyces cerevisiae Zellen Zellen des Stammes CEN.EN27 sind.

6. Verfahren nach einem der Ansprüche 1 bis 5, **dadurch gekennzeichnet, dass** das YGR046w Gen durch homologe Rekombination aus der oder den Saccharomyces cerevisiae Zellen entfernt und durch ein Markergen ersetzt wurde.

7. Verfahren nach Anspruch 6, wobei das Gen YGR046w im Genom von Saccharomyces cerevisiae mit Hilfe eines der Plasmide pPK5/6 (gemäß SEQ ID NO. 18), pPK7/8 (gemäß SEQ ID NO. 19), pPK9/10 (gemäß SEQ ID NO. 20) oder pPK13/14 (gemäß SEQ ID NO. 21) gegen ein Markergen ausgetauscht wurde.

8. Verfahren gemäß einem der vorstehenden Ansprüche, **dadurch gekennzeichnet, dass** der rekombinante Expressionsvektor in der Zelle in hoher Kopienzahl vorliegt.

9. Verfahren gemäß einem der vorstehenden Ansprüche, **dadurch gekennzeichnet, dass** die Stärke der Expression des YGR046w Gens durch die Art des regulierbaren Promotors und/oder die Wahl des Kulturmediums beeinflusst wird.

## Claims

1. A process for finding antimycotically active substances, which comprises providing at least one Saccharomyces cerevisiae cell which overexpresses, in relation to comparison cells, the gene YGR046w owing to transformation with a recombinant expression vector which comprises the coding part of the YGR046w gene under the control of a regulatable promoter, incubating this cell with a substance to be investigated, and determining the growth-inhibiting effect of this substance.

2. The process as claimed in claim 1, where at least two Saccharomyces cerevisiae cells which express the gene YGR046w to differing extents are provided, these cells are incubated with a substance to be investigated, and the growth-inhibiting effect of this substance is determined in a comparative manner.

3. The process as claimed in either of claims 1 to 2, where the cells are Saccharomyces cerevisiae cells of the strain CEN.PK2 or derivatives thereof.

4. The process as claimed in any of claims 1 to 3, where the cells are haploid Saccharomyces cerevisiae cells.

5. The process as claimed in either of claims 3 or 4, where the Saccharomyces cerevisiae cells are cells of the strain CEN.EN27.

6. The process as claimed in any of claims 1 to 5, wherein the YGR046w gene has been deleted by homologous recombination from the Saccharomyces cerevisiae cell(s) and has been replaced by a marker gene.

7. The process as claimed in claim 6, where the gene YGR046w in the genome of Saccharomyces cerevisiae has been replaced by a marker gene with the aid of one of the plasmids pPK5/6 (in accordance with SEQ ID NO:18), pPK7/8 (in accordance with SEQ ID NO:19), pPK9/10 (in accordance with SEQ ID NO:20) or pPK13/14 (in accordance with SEQ ID NO: 21).

8. The process as claimed in any of the preceding claims, wherein the recombinant expression vector is present in the cell in a high copy number.

9. The process as claimed in any of the preceding claims, wherein the strength of expression of the YGR046w gene is influenced by the nature of the regulatable promoter and/or the choice of the culture medium.

## Revendications

1. Procédé pour le criblage de substances à activité antimycosique, **caractérisé en ce qu'**on produit au moins une cellule de *Saccharomyces cerevisiae* qui surexprime le gène YGR046w, par rapport à des cellules comparatives, en raison de la transformation par un vecteur d'expression recombinant qui contient la partie codante du gène YGR046w sous le contrôle d'un promoteur régulable, on met cette cellule à incuber avec une substance à étudier et on détermine l'activité inhibitrice de croissance de cette substance.

2. Procédé selon la revendication 1, dans lequel on produit au moins deux cellules de *Saccharomyces cerevisiae* qui expriment à un degré différent le gène YGR046w, on met ces cellules à incuber avec une substance à étudier et on détermine par comparaison l'activité inhibitrice de croissance de cette substance.

3. Procédé selon la revendication 1 ou 2, dans lequel les cellules sont des cellules de *Saccharomyces cerevisiae* de la souche CEN.PK2 ou des dérivés de celle-ci.

4. Procédé selon l'une quelconque des revendications 1 à 3, dans lequel les cellules sont des cellules haploïdes de *Saccharomyces cerevisiae*.

5. Procédé selon la revendication 3 ou 4, dans lequel les cellules de *Saccharomyces cerevisiae* sont des cellules de la souche CEN.EN27.

6. Procédé selon l'une quelconque des revendications 1 à 5, **caractérisé en ce que** le gène YGR046w a été éliminé de la cellule ou des cellules de *Saccharomyces cerevisiae* par recombinaison homologue et remplacé par un gène marqueur.

7. Procédé selon la revendication 6, dans lequel le gène YGR046w dans le génome de *Saccharomyces cerevisiae* a été échangé contre un gène marqueur à l'aide d'un des plasmides pPK5/6 (selon SEQ ID n° 18), pPK7/8 (selon SEQ ID n° 19), pPK9/10 (selon SEQ ID n° 20) ou pPK13/14 (selon SEQ ID n° 21).

8. Procédé selon l'une quelconque des revendications précédentes, **caractérisé en ce que** le vecteur d'expression recombinant est présent en un grand nombre de copies dans la cellule.

9. Procédé selon l'une quelconque des revendications précédentes, **caractérisé en ce que** l'intensité de l'expression du gène YGR046w est influencée par la nature du promoteur régulable et/ou le choix du milieu de culture.
